# EUROPEAN PATENT SPECIFICATION

(11) **EP 3 787 636 B1**
(45) Date of publication and mention of the grant of the patent: **04.11.2026**
(21) Application number: 19723699.5
(22) Date of filing: 30.04.2019
(51) Int. Cl.: A61K 31/663, A61P 29/00

(54) **METHODS OF TREATING COMPLEX REGIONAL PAIN SYNDROME (CRPS) OR SYMPTOMS COMPRISING ADMINISTRATION OF NERIDRONIC ACID**
VERFAHREN ZUR BEHANDLUNG VON KOMPLEXEM REGIONALEM SCHMERZSYNDROM (CRPS) ODER SYMPTOMEN MIT VERABREICHUNG VON NERIDRONSÄURE
MÉTHODES DE TRAITEMENT DU SYNDROME DE DOULEUR RÉGIONALE COMPLEXE (SDRC) OU DE SYMPTÔMES COMPRENANT L'ADMINISTRATION D'ACIDE NÉRIDRONIQUE

(30) Priority: 30.04.2018 US 201862664496 P
(43) Date of publication of application: 10.03.2021
(73) Proprietor: Abiogen Pharma SpA, 56121 Ospedaletto (PISA) (IT)
(72) Inventor: LANGE, Bernd, 41836 Hückelhoven (DE); BENAY, Stephan, 52078 Aachen (DE); EERDEKENS, Marielle, 3650 Dilsen-Stokkem (BE); ENGELEN, Sylvia, 6294 NM Vijlen (NL); THUN, Susanne, 52078 Aachen (DE); KNEIP, Christa, 52080 Aachen (DE)
(74) Representative: Villa, Livia
(86) International application number: PCT/EP2019/061003
(87) International publication number: WO 2019/211261

(56) References cited:
- VARENNA M ET AL: "Treatment of complex regional pain syndrome type I with neridronate: a randomized, double-blind, placebo-controlled study", RHEUMATOLOGY, OXFORD UNIVERSITY PRESS, UNITED KINGDOM, UNITED STATES, NETHERLANDS, vol. 52, no. 3, 1 March 2013 (2013-03-01), pages 534 - 542, XP002734047, ISSN: 1462-0332, [retrieved on 20121130], DOI: 10.1093/RHEUMATOLOGY/KES312
- GEOFFREY LITTLEJOHN: "Bisphosphonates for early complex regional pain syndrome", NATURE REVIEWS RHEUMATOLOGY, vol. 9, no. 4, 29 January 2013 (2013-01-29), GB, pages 199 - 200, XP055606660, ISSN: 1759-4790, DOI: 10.1038/nrrheum.2013.6
- JIJUN XU ET AL: "Intravenous Therapies for Complex Regional Pain Syndrome : A Systematic Review", ANESTHESIA AND ANALGESIA., vol. 122, no. 3, 1 March 2016 (2016-03-01), US, pages 843 - 856, XP055606662, ISSN: 0003-2999, DOI: 10.1213/ANE.0000000000000999
- CHEVREAU MAXIME ET AL: "Bisphosphonates for treatment of Complex Regional Pain Syndrome type 1: A systematic literature review and meta-analysis of randomized controlled trials versus placebo", JOINT BONE SPINE, ELSEVIER, AMSTERDAM, NL, vol. 84, no. 4, 11 April 2017 (2017-04-11), pages 393 - 399, XP085107868, ISSN: 1297-319X, DOI: 10.1016/J.JBSPIN.2017.03.009
- MARK A. PERAZELLA ET AL: "Bisphosphonate nephrotoxicity", KIDNEY INTERNATIONAL, vol. 74, no. 11, 1 December 2008 (2008-12-01), LONDON, GB, pages 1385 - 1393, XP055606997, ISSN: 0085-2538, DOI: 10.1038/ki.2008.356
- J F MAILLEFERT ET AL: "Treatment of refractory reflex sympathetic dystrophy with pamidronate.", ANNALS OF THE RHEUMATIC DISEASES, vol. 54, no. 8, 1 August 1995 (1995-08-01), GB, pages 687 - 687, XP055607075, ISSN: 0003-4967, DOI: 10.1136/ard.54.8.687-a
- YANOW J ET AL: "Complex regional pain syndrome (CRPS/RSD) and neuropathic pain: Role of intravenous bisphosphonates as analgesics", THE SCIENTIFIC WORLD JOU, THE SCIENTIFICWORLD LTD, GB, NL, vol. 8, 25 February 2008 (2008-02-25), pages 229 - 236, XP009148122, ISSN: 1537-744X, DOI: 10.1100/TSW.2008.33

## Description

### Cross-Reference to Related Applications

This application claims priority to U.S. Provisional Application No. 62/664,496, filed April 30, 2018.

### Field of the Invention

The invention is directed to neridronic acid for use in a method of treating complex regional pain syndrome (CRPS) or symptoms, such as pain associated with CRPS wherein neridronic acid is administered intravenously.

### Background of the Invention

Complex regional pain syndrome is a rare, but severely disabling pain syndrome characterized by spontaneous and evoked regional pain, usually beginning in a distal extremity, that is disproportionate in magnitude or duration to the typical course of pain after similar tissue trauma (Bruehl S BMJ 2015; 350:h2730).

The disease is usually triggered by an injury, but spontaneous-onset cases of CRPS, with a similar clinical picture, are nevertheless possible. The development of the disease is not linked to the severity of trauma. Fractures (about 45 %), sprain (about 18 %), and elective surgery (about 12 %) are the most frequently reported triggering events (Gatti D, Rossini M, Adami S Osteoporos Int 2016; 27: 2423).

The syndrome includes a wide range of conditions affecting a peripheral limb characterized by chronic disabling pain that is seemingly disproportionate in time or degree to the usual course of any known trauma or other lesion. The pain is regional, and it is typically associated with abnormal sensory, motor, sudomotor, vasomotor, and/or trophic findings (Gatti, 2016). Limbs of affected patients may display extreme hyperalgesia and allodynia to normally non-painful stimuli such as draft, touch or cold. Changes in skin color, skin temperature, and sweating behaviors relative to the unaffected collateral limb, edema and altered patterns of hair, skin, or nail growth in the affected region as well as reduced strength; tremors; and dystonia are further sign and symptoms (Bruehl, 2015).

Therefore, CRPS is often associated with serious impairment of daily life activities.

The pathophysiology of CRPS is still poorly understood (Gatti, 2016) and the available diagnostic criteria for CRPS rely on clinical criteria (Harden RN, Bruehl S, Galer SR, Pain 1999; 83: 211-9; Harden RN et al. Pain 2010; 150: 268-274). As currently conceptualized, CRPS is subdivided into type I and type II (CRPS-I and CRPS-II) based on the absence or presence, respectively, of clinical signs of major peripheral nerve injury (such as nerve conduction study abnormalities). Despite this clinical distinction, core diagnostic features are identical across both subtypes (Bruehl, 2015). Furthermore, there is a symptomatic differentiation of CRPS into warm CRPS, exhibiting an increased skin temperature at the onset of CRPS ("inflammation type") and into cold CRPS, exhibiting a decreased skin temperature at the onset (Birklein F, O'Neill D, Schlereth T, Neurology 2015; 84: 89-96).

Limited data are available on the incidence of CRPS, and the disease is underestimated and under-diagnosed. The disease shows a female preponderance of approximately 3:1 with a peak age of incidence between about 50 to 60 years.

Effective management of the syndrome is often challenging. Currently, there is no FDA approved treatment for CRPS available. Furthermore, few high quality randomized controlled trials are available to support the efficacy of the most commonly used interventions.

There is some indication that therapeutic prognosis for patients with the warm subtype of CRPS is better than for patients with cold CRPS (Gatti, 2016; Birklein, 2015). Likewise, patients with a fracture as the predisposing event or patients in an early stage of the disease appear to have an increased prognosis for a positive treatment response. Responder patients showed a disease duration significantly shorter in comparison with nonresponders (median = 3 months, inter quartile range (IQR) = 2-5; vs median = 5 months, IQR = 3-8). (Varenna M, Manara M, Rovelli F, Zucchi F, Sinigaglia L Pain Medicine 2017; 18: 1131-1138).

One treatment suggestion involves the application of bisphosphonate compounds (Gatti, 2016). Several bisphosphonate compounds, such as neridronic acid, have been successfully tested clinically for the treatment of various conditions associated with Complex Regional Pain Syndrome (Varenna M et al., Rheumatology 2013; 52: 534-542).

Nephrotoxicity is a known consequence of dosing with bisphosphonates (Pfister T, Atzpodien E, Bohrmann B, Frieder B Acta Pharm Toxicol 2005; 97(6): 374-815). In animal studies renal tubular degeneration and regeneration was identified as the primary mechanism. Dose and infusion time are the main influencing factors related to renal toxicity (Perazella MA, Markowitz GS, Kidney Int 2008; 74(11): 1385-93).Littlejohn G. "Bisphosphonates for early complex regional pain syndrome", NATURE REVIEWS RHEUMATOLOGY, vol. 9, no. 4, 29 January 2013 (2013-01-29), pages 199-200, discloses that bisphosphonates are useful for early complex regional pain syndrome, in 100mg doses of neridronate intravenously four times over 10 days.
JIJUN XU ET AL: "Intravenous Therapies for Complex Regional Pain Syndrome : A Systematic Review", ANESTHESIA AND ANALGESIA, vol. 122, no. 3, 1 March 2016 (2016-03-01), pages 843-856, discloses that 100mg of intravenous infusion of neridronate given 4 times over 10 days is useful for Complex Regional Pain Syndrome. CHEVREAU MAXIME ET AL: "Bisphosphonates for treatment of Complex Regional Pain Syndrome type 1: A systematic literature review and meta-analysis of randomized controlled trials versus placebo", JOINT BONE SPINE, ELSEVIER, vol. 84, no. 4, 11 April 2017 (2017-04-11), pages 393-399, disclose that 100mg neridronate are given i.v. four times in 10 days to treat Complex Regional Pain Syndrome. J F MAILLEFERT ET AL: "Treatment of refractory reflex sympathetic dystrophy with pamidronate", ANNALS OF THE RHEUMATIC DISEASES, vol. 54, no. 8, 1 August 1995 (1995-08-01), pages 687-687, evaluated the efficacy of pamidronate in the treatment of refractory RSD. YANOW J ET AL: "Complex regional pain syndrome (CRPS/RSD) and neuropathic pain: Role of intravenous bisphosphonates as analgesics", THE SCIENTIFIC WORLD JOU, THE SCIENTIFICWORLD LTD, GB, NL, vol. 8, 25 February 2008 (2008-02-25), pages 229-236, the preclinical studies were reviewed regarding the use of bisphosphonates as analgesics in animal models of neuropathic pain, and also summarized the clinical trials that have been done to date. The authors gave an overview of bisphosphonate pharmacology and discuss several potential mechanisms by which bisphosphonates may be analgesic in CRPS/RSD and bone pain of noncancer origin.

Bisphosphonates may cause renal toxicity by tubular injury.

Cellular toxicity *in vitro* depends on both concentration and duration of exposure (Verhulst A et al. PLoS One. 2015;10(3):e0121861.). Consequently, one may anticipate that both, Cₘₐₓ (maximum systemic concentration) and AUC (area under the plasma concentration curve) determine kidney liabilities in vivo.

The bisphosphonate concentrations in the kidney will depend on both, cellular uptake and the rate of elimination. Different renal tissue half-lives have been reported for different bisphosphonates. A short renal tissue half-life may avert accumulation by producing lower steady-state concentrations in renal cells. However, the renal tissue half-life of neridronic acid has not been established and there is no firm evidence for different tissue elimination phase half-lives of bisphosphonates. Moreover, exposure of bisphosphonates appears to be biphasic with most of the compound being eliminated during the first 24 h, followed by a much slower beta-phase.

Renal cell uptake of bisphosphonates *in vitro* is non-saturable, thus also depending on both, concentration and duration of exposure. Nonclinical toxicology studies with neridronic acid do not allow conclusions regarding the impact of infusion time on renal toxicity because the study designs are too different from each other and the question has not been investigated in a designated study.

Even though *in vivo* both AUC and Cₘₐₓ appear to determine the renal tolerability of bisphosphonates, clinically, renal complications have been mostly but not exclusively reported with IV (intravenously administered) bisphosphonates, which is consistent with the greater albeit transient exposure of the kidneys to drug. AKI (acute kidney injury) has been reported with IV etidronate, orally administered clodronate, IV pamidronate, orally administered alendronate, oral risedronate, IV ibandronate, and IV zoledronic acid. (Hirschberg R Curr Opin Support Palliat Care. 2012;6(3):342-7).

Thus, human clinical data showing that renal effects are primarily seen with IV bisphosphonates suggest an influence of Cₘₐₓ on nephrotoxicity. For neridronic acid, however, an appropriate infusion time analysis has not been done or reported. Thus, it remains a need for a method to safely and efficaciously administer neridronic acid to a patient suffering from CRPS, especially for those patients with a reduced or impaired renal function.

### Brief Summary

This invention is directed to a method of treating CRPS and/or treating symptoms associated with CRPS by intravenously administering to a subject in need of treatment of CRPS or of one or more symptoms associated with CRPS an effective amount for treating CRPS or the one or symptoms associated with CRPS of neridronic acid. The scope of the invention is defined by the claims. Any references in the description to methods of treatment refer to the compounds, pharmaceutical compositions and medicaments of the present invention for use in a method for treatment of the human (or animal) body by therapy.

Furthermore, the method according to the invention is particularly suitable to increase the safety margin for patients, especially for those with a reduced or impaired renal function.

### Figures

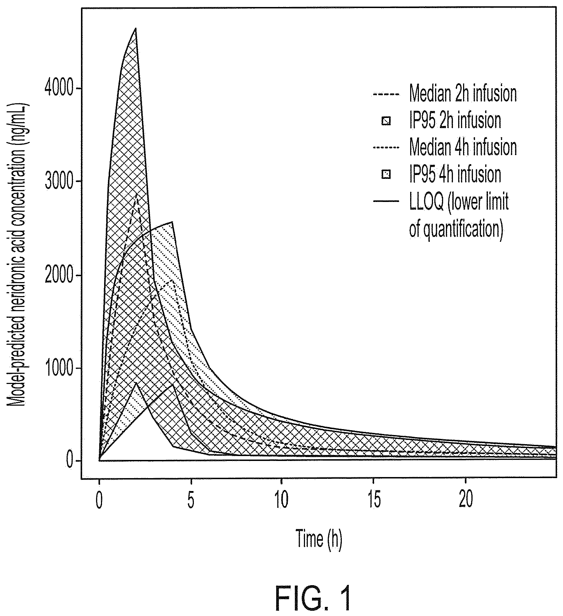

Figure 1 shows the final PopPK model-predicted concentrations of neridronic acid in the central compartment after a 2 h or 4 h infusion of 100 mg of neridronic acid at day 1.

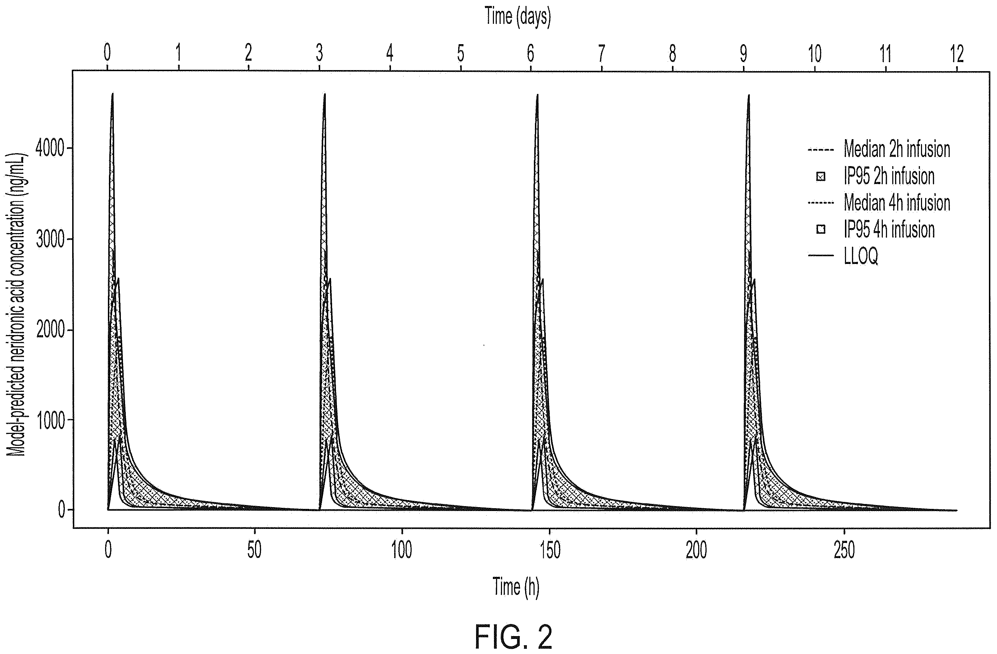

Figure 2 shows the final PopPK model-predicted concentrations of neridronic acid in the central compartment after 2 h or 4 h infusions of 100 mg of neridronic acid (cumulative dose of 400 mg) at days 1, 4, 7, and 10.
Figure 3 shows the pain decrease depending on the duration of CRPS with a mean change from baseline with 95% confidence interval on the NRS score. Clinically relevant pain decrease is shown in the subgroup of patients suffering up to 2 years from CRPS at the primary endpoint (day 84) with a mean reduction of -2.29.

### Detailed Description of the Invention

A first aspect of the invention relates to neridronic acid for use in a method of treating complex regional pain syndrome (CRPS) or pain associated with CRPS, which comprises administering to a subject in need thereof an therapeutically effective amount of neridronic acid, wherein the therapeutically effective amount of neridronic acid is administered intravenously in one or in two or more individual unit doses, wherein each individual dose of neridronic acid is administered to the subject by intravenous infusion over a duration of at least 3 hours, at least 4 hours or at least 5 hours.

One embodiment of the first aspect of the invention relates to
neridronic acid for use in a method of treating complex regional pain syndrome (CRPS) or pain associated with CRPS,
which comprises administering to a subject in need thereof an therapeutically effective amount of neridronic acid,
characterized in that
the effective amount of neridronic acid is administered intravenously in one or in two or more individual unit doses, and
each dose of neridronic acid is administered to the subject by intravenous infusion over a duration of at least 3 hours, at least 4 hours or at least 5 hours.

### Definitions

The term "treating" or "treatment" includes any kind of treatment activity, including the diagnosis, cure, mitigation, or prevention of disease in subjects such as man or mammal, or any activity that otherwise affects the structure or any function of the body of subjects such as man or mammal.

The term "method" or "method of treatment" is used herein synonymously to the term "method for treatment of CRPS or pain associated with CRPS" unless expressly defined differently.

The term "neridronic acid" relates to all forms of the free acid and its solvates (e.g., hydrates), physiologically acceptable salts thereof ("neridronate"), alternate solid forms (e.g., polymorphs, molecular complexes), as well as any other chemical species that may rapidly convert to neridronic acid under conditions in which neridronic acid is used as described herein. In particular, the term "neridronic acid" relates to the free acid, a physiologically acceptable salt thereof, and a solvate of the free acid or of a physiologically acceptable salt thereof. Any amount of neridronic acid given herein always refers to the amount of the free acid.

Preferred salts of neridronic acid are its alkali metal salts, particularly preferred are sodium salts of neridronic acid (sodium neridronate). Most preferred is mono sodium neridronate.

Preferred solvates of neridronic acid are hydrates, including but not limited to hemi-hydrates.

A composition or dosage form comprising neridronic acid is defined as a composition suitable for intravenous administration comprising an effective amount of neridronic acid.

In a preferred embodiment, the dosage form comprising neridronic acid is an aqueous solution comprising neridronic acid and optionally further excipients that are suitable for intravenous administration.

The term "intravenous" is defined as within or administered into a vein of subject.

Complex regional pain syndrome (CRPS) is also known as Causalgia, Trophoneurosis, Acute bone atrophy, Sudeck atrophy, Post-traumatic painful osteoporosis, Acute peripheral neuroatrophy, Traumatic angiospasm, Post-traumatic osteoporosis, Traumatic vasospasm, Reflex limb dystrophy, Minor and major causalgia, Post-infarction sclerodactyly, Shoulder-hand syndrome, Algodystrophy, Osteodystrophy, Causalgia minor, Algo-neuro-dystrophy, Sympathetic sustained pain syndromes, Sympathetic dependent pain, Hyperactive sympathetic syndrome, Reflex sympathetic dystrophies (RSD) or Complex regional pain disease (CRPD) (Gatti, 2016). The term "Complex regional pain syndrome" is herein understood as synonymous for the above terms.

Symptoms of CRPS are herein defined as pain, abnormal sensory findings, like hyperesthesia, hyperalgesia or allodynia, vasomotor findings, like temperature asymmetry, skin colour changes, or skin colour asymmetry, sudomotor findings, like oedema, sweating changes, or sweating asymmetry, motoric findings, like decreased range of motion, or motor dysfunction such as weakness, tremor, or dystonia, and trophic changes, such as in hair, nails, or skin.

In some embodiments, the inventive method is related to the treatment of complex regional pain syndrome type I (CRPS-I) or one or more symptoms associated with complex regional pain syndrome type I. In some embodiments, the inventive method is related to the treatment of pain associated with complex regional pain syndrome type I.

In some embodiments, the inventive method is related to the treatment of complex regional pain syndrome type II (CRPS-II) or one or more symptoms associated with complex regional pain syndrome type II. In some embodiments, the inventive method is related to the treatment of pain associated with complex regional pain syndrome type **II.**

In some embodiments, the inventive method is related to the treatment of CRPS-I and CRPS-II or one or more symptoms associated with CRPS I and CRPS-II. In some embodiments, the inventive method is related to the treatment of pain associated with CRPS I and CRPS-II.

In some embodiments, the inventive treatment method is independent on the subtype of CRPS with regard to warm or cold CRPS, so no differentiation between warm CRPS and cold CRPS is identified in the response rate of the treatment.

In some embodiments, the inventive treatment method is independent on the predisposing event, so no differentiation between patients with different predisposing events, such as a fracture, is identified in response rate of the treatment.

The inventive method of treating complex regional pain syndrome (CRPS) is herein understood as treatment of CRPS itself as well as treatment of the symptoms of CRPS as defined above. In particular, the treatment of CRPS includes the treatment of pain associated with CRPS:

In one embodiment, the treatment for pain associated with CRPS is the relief of pain associated with CRPS as well as all changes, improvements or reductions in the symptoms of CRPS that improve the quality of life of the treated subject.

Improvements in the quality of life may include improved mobility and activity in the daily life of the treated subject.

For this invention, the term "subject" refers to a mammal, e.g., rat, mouse, dog, monkey, or a human being, particularly preferred to a human being.

In some embodiments, a human being that is treated for CRPS or pain associated with CRPS by neridronic acid, e.g., by administration of a dosage form of neridronic acid, has an age of at least 18 years, at least 40 years, at least 50 years, about 10 years to about 90 years, about 20 years to about 80 years, about 30 years to about 75 years, about 40 years to about 70 years, about 45 years to about 65 years, or about 50 years to about 60 years.

The terms "effective amount" or "therapeutically effective amount" are generally understood as an amount of the agent (in this case, neridronic acid) at least sufficient to provide the desired therapeutic effect. (Preferably, nontoxic levels of the active agent will be employed, if possible). The exact amount required will vary from subject to subject, depending on age, general condition of the subject, the severity of the condition being treated, and the like. Thus, herein, the terms "effective amount" or "therapeutically effective amount" are defined as the amount of the agent which is sufficient to provide the desired average therapeutic effect in all subjects or in a specific subgroup of subjects.

In some embodiments, the effective amount of neridronic acid employed in the present invention ranges from between about 0.25 mg and about 25 mg of neridronic acid/kg of body weight of the subject, preferably between about 0.5 mg and about 20 mg of neridronic acid/kg of body weight of the subject, preferably between about 1.0 mg and about 12 mg of neridronic acid/kg of body weight of the subject, preferably between about 1.5 mg and about 10 mg of neridronic acid/kg of body weight of the subject, preferably between about 2.5 mg and about 7.5 mg of neridronic acid/kg of body weight of the subject, and is most preferably about 6.0 mg of neridronic acid/kg of body weight of the subject.

In some embodiments, the effective amount of neridronic ranges from about 20 mg to about 1500 mg, from about 25 mg to about 1400 mg, from about 30 mg to about 1250 mg, from about 40 mg to about 1000 mg, from about 50 mg to about 900 mg, from about 60 mg to about 850 mg, from about 75 mg to about 800 mg, from about 100 mg to about 750 mg, from about 125 mg to about 720 mg, from about 150 mg to about 700 mg, from about 180 mg to about 650 mg, from about 200 mg to about 600 mg, from about 250 mg to about 550 mg, from about 280 mg to about 520 mg, from about 300 mg to about 500 mg, and preferably from about 350 mg to about 450 mg.

In some embodiments, the effective amount of neridronic is about 600 +/- 400 mg, about 600 +/- 300 mg, about 600 +/- 200 mg, about 500 +/- 400 mg, about 500 +/-250 mg, about 500 +/- 200 mg, about 500 +/- 120 mg, about 450 +/- 300 mg, about 450 +/- 250 mg, about 450 +/- 200 mg, about 450 +/- 120 mg, about 450 +/- 100 mg, about 450 +/- 80 mg, about 450 +/- 60 mg, about 450 +/- 50 mg, about 400 +/- 200 mg, about 400 +/- 150 mg, about 400 +/- 120 mg, about 400 +/- 100 mg, about 400 +/- 80 mg, about 400 +/- 60 mg, about 400 +/- 40 mg, about 320 +/- 200 mg, about 320 +/- 160 mg, about 320 +/- 120 mg, about 320 +/- 100 mg, about 320 +/- 80 mg, about 320 +/- 60 mg, about 320 +/- 40 mg, about 250 +/- 150 mg, about 250 +/- 125 mg, about 250 +/- 100 mg, about 250 +/- 75 mg, about 250 +/- 50 mg, about 250 +/- 40 mg, about 250 +/- 25 mg, about 150 +/- 75 mg, about 150 +/- 60 mg, about 150 +/- 50 mg, about 150 +/- 40 mg, about 150 +/- 30 mg, about 150 +/- 25 mg, about 150 +/- 15 mg.

The effective amount of neridronic acid can be administered in one or in two or more individual unit doses. Some embodiments include treatment of CRPS or pain associated with CRPS, wherein the treatment comprises administering a first dosage form to a subject, followed by administering of a second dosage form or multiple dosage forms to the subject. In some embodiments, a dosage form comprising neridronic acid is administered every 1, 2, 3, 4, 5, 6, 7, 8, 9, 10, 11, 12, 13, or 14 days; or 15, 16, 17, 18, 19, 20, or 21 days.

The method of the invention involves administration of the effective amount of neridronic acid in one or more separate unit doses that can be administered to the patient anywhere from a day to a month apart. In some embodiments, the second unit dose (or each consecutive unit dose) is administered about 12 hours to about 28 days, about 24 hours to about 21 days, about 24 hours to about 14 days, about 24 hours to about 7 days, or about 24 hours to about 3 days, after the first unit dose (or the preceding unit dose) is administered. In some embodiments, the intervals between the administrations of each unit dose are equal.

In some embodiments, the therapeutically effective amount of neridronic acid is administered in 2 or 3 or 4 or 5 or 6 doses within 2 months, within 1 month, within 4 weeks, within 3 weeks, within 2 weeks, within 1 week, within 14 days, within 12 days, within 10 days, within 8 days, within 7 days, within 6 days, within 4 days, within 3 days or within 2 days.

In some embodiments, the therapeutically effective amount of neridronic acid is administered in 2 or 3 or 4 or 5 unit doses within less than 3 weeks, preferably less than 2 weeks, wherein the intervals between the administrations of each unit dose are preferably equal.

In some embodiments, the therapeutically effective amount of neridronic acid is administered in 2 unit doses with an interval of 2 days or 3 days or 4 days between each administration, resulting in an administration on day 1 and day 3, or an administration on day 1 and day 4, or an administration on day 1 and day 5, respectively.

In some embodiments, the therapeutically effective amount of neridronic acid is administered in 3 unit doses with an interval of 2 days or 3 days or 4 days between each administration, resulting in an administration on day 1, day 3 and day 5, or an administration on day 1, day 4 and day 7, or an administration on day 1, day 5 and day 9, respectively.

In some embodiments, the therapeutically effective amount of neridronic acid is administered in 4 unit doses with an interval of 2 days or 3 days or 4 days between each administration, resulting in an administration on day 1, day 3, day 5 and day 7, or an administration on day 1, day 4, day 7 and day 10, or an administration on day 1, day 5, day 9 and day 13, respectively.

In some embodiments, the therapeutically effective amount of neridronic acid is administered in 5 unit doses with an interval of 2 days or 3 days or 4 days between each administration, resulting in an administration on day 1, day 3, day 5, day 7 and day 9, or an administration on day 1, day 4, day 7, day 10 and day 13, or an administration on day 1, day 5, day 9, day 13 and day 17, respectively.

To avoid any undesired side effects or adverse events, such as hepatic or renal damage, each unit dose is generally between about 0.1 mg and about 6 mg of neridronic acid/kg of body weight of the subject, between about 0.15 mg and about 5 mg of neridronic acid/kg of body weight of the subject, between about 0.2 mg and about 4 mg of neridronic acid/kg of body weight of the subject, between about 0.5 mg and about 3 mg of neridronic acid/kg of body weight of the subject, between about 1.0 mg and about 2.0 mg of neridronic acid/kg of body weight of the subject, preferably between about 1.2 mg and about 1.8 mg of neridronic acid/kg of body weight of the subject and most preferably about 1.5 mg of neridronic acid/kg of body weight of the subject.

**In** some embodiments, a preferred unit dose of neridronic acid ranges from about 10 mg to about 400 mg, from about 15 mg to about 300 mg, from about 25 mg to about 250 mg, from about 40 mg to about 240 mg, from about 50 mg to about 200 mg, from about 60 mg to about 180 mg, from about 75 mg to about 150 mg and most preferably from about 90 mg to about 120 mg.

**In** some embodiments, a preferred unit dose of neridronic acid is about 150 +/-100 mg, about 150 +/- 75 mg, about 150 +/- 50 mg, about 120 +/- 100 mg, about 120 +/-60 mg, about 120 +/- 40 mg, about 120 +/- 30 mg, about 100 +/- 75 mg, about 100 +/- 60 mg, about 100 +/- 50 mg, about 100 +/- 30 mg, about 100 +/- 25 mg, about 100 +/- 20 mg, about 100 +/- 15 mg, about 100 +/- 10 mg, about 80 +/- 50 mg, about 80 +/- 40 mg, about 80 +/- 30 mg, about 80 +/- 25 mg, about 80 +/- 20 mg, about 80 +/- 15 mg, about 80 +/- 10 mg, about 60 +/- 50 mg, about 60 +/- 40 mg, about 60 +/- 30 mg, about 60 +/-25 mg, about 60 +/- 20 mg, about 60 +/- 15 mg, about 60 +/- 10 mg.

In some embodiments, a preferred unit dose of neridronic acid is 150 mg, 120 mg, 100 mg, 80 mg, 75 mg, or 60 mg.

In one embodiment, the therapeutically effective amount of neridronic acid is about 150 mg or about 200 mg or about 250 mg or about 300 mg or about 400 mg or about 500 mg which is administered in 2 equal unit doses on day 1 and day 3, or on day 1 and day 4, or on day 1 and day 5.

In one embodiment, the therapeutically effective amount of neridronic acid is about 150 mg or about 200 mg or about 250 mg or about 300 mg or about 400 mg or about 500 mg which is administered in 3 equal unit doses on day 1, day 3 and day 5, or on day 1, day 4 and day 7, or on day 1, day 5 and day 9.

In one embodiment, the therapeutically effective amount of neridronic acid is about 150 mg or about 200 mg or about 250 mg or about 300 mg or about 400 mg or about 500 mg which is administered in 4 equal unit doses on day 1, day 3, day 5 and day 7, or on day 1, day 4, day 7 and day 10, or on day 1, day 5, day 9 and day 13.

In one embodiment, the therapeutically effective amount of neridronic acid is about 150 mg or about 200 mg or about 250 mg or about 300 mg or about 400 mg or about 500 mg which is administered in 5 equal unit doses on day 1, day 3, day 5, day 7 and day 9, or on day 1, day 4, day 7, day 10 and day 13, or on day 1, day 5, day 9, day 13 and day 17.

In a preferred embodiment, the therapeutically effective amount of neridronic acid is about 400 mg which is administered in 4 equal unit doses on day 1, day 4, day 7 and day 10.

Given that neridronic acid is almost completely eliminated via renal excretion, this is a major consideration in patients with renal impairment. After intravenous administration of a dosage form containing neridronic acid the capacity of the kidney to excrete neridronate should be considered in order to avoid adverse events affecting subjects, in particular for those subjects having renal impairment. Thus, it was necessary to optimize the duration of administration of the dosage to increase the renal safety margin for all subjects independent of renal impairment status and to obtain a sufficient safety margin for those subjects having renal impairment while keeping the administration as short as possible for any of the subjects, thus avoiding any negative influence on the compliance to the treatment by the subject.

In one aspect of the invention, the inventive method of treatment even increases the renal safety margin for all subjects independent of renal impairment status while keeping the administration time as short as possible for any of the subjects to avoid any negative influence on the compliance to the treatment by the subject.

According to the invention, each dose of neridronic acid is preferably administered to the subject by intravenous infusion over a duration of at least 3 hours, at least 4 hours or at least 5 hours. In some embodiments, a preferred duration of the intravenous administration of the dose of neridronic acid is about 180 min, about 210 +/-30 min, about 210 +/- 15 min, about 240 +/- 60 min, about 240 +/- 45 min, about 240 +/-40 min, about 240 +/- 30 min, about 240 +/- 20 min, about 240 +/- 15 min, about 270 +/-75 min, about 270 +/- 60 min, about 270 +/- 45 min, about 270 +/- 40 min, about 270 +/-30 min, about 270 +/- 20 min, about 270 +/- 15 min, about 300 +/- 120 min, about 300 +/-90 min, about 300 +/- 75 min, about 300 +/- 60 min, about 300 +/- 45 min, about 300 +/-30 min, preferably about 240 +/- 60 min, about 240 +/- 30 min, about 240 +/- 15 min, about 300 +/- 120 min, about 300 +/- 90 min, about 300 +/- 60 min or about 300 +/- 30 min.

In some embodiments, the inventive method results in a reduced maximum systemic concentration of neridronic acid (Cₘₐₓ) which may result in an improved renal safety margin for acute renal damage of subjects having renal impairment.

The term "maximum concentration" or "Cₘₐₓ" is defined as the peak concentration that a drug achieves in a specified compartment after the drug has been administrated and before administration of a second dose. Herein, Cₘₐₓ is defined as peak mass concentration of neridronic per volume blood in the blood system of the subject.

Adverse events, like acute phase reactions (polyarthralgia and/or fever and/or even acute renal damage; Adami S, Bhalla AK, Dorizzi R, Montesanti F, Rosini S, Salvagno G, Lo Cascio V, Cacif Tissue Int 1987, 41: 326-331), are more likely to occur at concentration levels that are above the NOAEL (No adverse events level). In some embodiments, the NOAEL is about 2500 +/- 250 ng/mL, 3000 +/- 250 ng/mL, about 3250 +/- 250 ng/mL, about 3500 +/- 250 ng/mL, about 4000 +/- 250 ng/mL. In a preferred embodiment, the NOEAL is about 3250 +/- 250 ng/mL.

In some embodiments, the inventive method provides a Cₘₐₓ of neridronic acid of about 100 ng/mL to about 3,500 ng/mL, about 250 ng/mL to about 3,200 ng/mL, about 500 ng/mL to about 3,000 ng/mL, about 750 ng/mL to about 2,750 ng/mL, about 1,000 ng/mL to about 2,500 ng/mL, about 1,250 ng/mL to about 2,250 ng/mL, about 1,500 ng/mL to about 2,000 ng/mL,
or any Cₘₐₓ in a range bounded by, or between, any of these values, upon administration of neridronic acid to a subject.

In some embodiments, the inventive method provides a Cₘₐₓ of neridronic acid of
about 1,850 +/- 750 ng/mL, about 1,850 +/- 600 ng/mL, ng/mL, about 1,850 +/- 500 ng/mL, preferably of about 1,850 +/- 500 ng/mL, or
about 1,200 +/- 750 ng/mL, about 1,200 +/- 600 ng/mL, ng/mL, about 1,200 +/- 500 ng/mL, preferably of about 1,200 +/- 500 ng/mL, or
about 1,500 +/- 750 ng/mL, about 1,500 +/- 600 ng/mL, ng/mL, about 1,500 +/- 500 ng/mL, preferably of about 1,500 +/- 500 ng/mL, or
about 1,750 +/- 750 ng/mL, about 1,750 +/- 600 ng/mL, ng/mL, about 1,750 +/- 500 ng/mL, preferably of about 1,750 +/- 500 ng/mL, or
about 2,000 +/- 750 ng/mL, about 2,000 +/- 600 ng/mL, ng/mL, about 2,000 +/- 500 ng/mL, preferably of about 2,000 +/- 500 ng/mL, or
about 2,500 +/- 750 ng/mL, about 2,500 +/- 600 ng/mL, ng/mL, about 2,500 +/- 500 ng/mL, preferably of about 2,500 +/- 750 ng/mL, or
about 3,000 +/- 1,250 ng/mL, about 3,000 +/- 750 ng/mL, ng/mL, about 3,000 +/- 500 ng/mL, preferably of about 3,000 +/- 1,250 ng/mL.

The renal safety margin is particularly important for patients who provide evidence for renal impairment. Renal impairment can be determined by an estimated glomerular filtration rate [eGFR] less than 30 mL/min/1.73 m² using the 2009 Chronic Kidney Disease Epidemiology Collaboration [CKD-EPI] creatinine equation (Levey AS et al. Ann Intern Med. 2009; 150(9): 604-612.] or by a urinary albumin to creatinine ratio [ACR] greater than 150 mg/g. Subjects with mild renal impairment are defined as having an eGFR of 60 to less than 90 mL/min/1.73 m². Subjects with moderate renal impairment are defined as having an eGFR of 30 to less than 60 mL/min/1.73 m², in particular either 45 to less than 60 mL/min/1.73 m² or 30 to less than 45 mL/min/1.73 m². Subjects having an eGFR of greater than 90 mL/min/1.73 m² are considered to have no renal impairment.

In some embodiments, the inventive method of treatment is suitable for all patients, with or without renal impairment. Treatment of patients without renal impairment may be preferred.

In some embodiments, the inventive method of treatment results in a higher renal safety margin for acute renal damage in the subject with a reduced or impaired renal function than it would be if each individual unit dose of neridronic acid were administered to the subject by intravenous infusion over a duration of less than 4 hours.

In some embodiments, the amount of neridronic acid used in the method of treatment is lowered for patients with renal impairment to increase the renal safety margin. In one embodiment, the total amount of neridronic acid in the treatment of patients with an eGFR of 45 to less than 60 mL/min/1.73 m² may be reduced to about 75 wt-% of the amount of neridronic acid used for patients with no or only mild renal impairment. In another embodiment, the total amount of neridronic acid in the treatment of patients with an eGFR of 30 to less than 45 mL/min/1.73 m² may be reduced to about 62.5 wt-% of the amount of neridronic acid used for patients with no or only mild renal impairment.

The renal safety margin is defined as the ratio of NOAEL over Cₘₐₓ. The higher the ratio of NOAEL over Cₘₐₓ, the higher is the renal safety margin during the treatment and the lower is the likelihood for adverse events.

Therefore, the inventive method of treatment may be different depending on the renal impairment situation of each patient:

For example, for subjects with no or mild renal impairment, the full contents of a single vial (8 mL, corresponding to 100 mg neridronic acid) may be diluted in 500 mL normal saline and administered by slow intravenous infusion at day 1, day 4, day 7 and day 10, resulting in a total dose of 400 mg neridronic acid,
for subjects with moderate renal impairment and an eGFR of 45 to less than 60 mL/min/1.73 m², 6 mL of the solution from a single vial (corresponding to 75 mg of neridronic acid) may be diluted in 500 mL normal saline and administered by slow intravenous infusion at day 1, day 4, day 7 and day 10, resulting in a total dose of 300 mg neridronic acid; and
for subjects with moderate renal impairment and an eGFR of 30 to less than 45 mL/min/1.73 m², 5 mL of solution from a single vial (corresponding to 62.5 mg of neridronic acid) may be diluted in 500 mL normal saline and administered by slow intravenous infusion at day 1, day 4, day 7 and day 10, resulting in a total dose of 250 mg neridronic acid.

In some embodiments, the inventive method provides a ratio of NOAEL over Cₘₐₓ of neridronic acid that is about 1.2, about 1.3, about 1.5, about 1.75, about 2, about 2.5, about 3, about 3.5, or about 5.

The difference between Cₘₐₓ and NOAEL may be used as another measure for renal safety margin; the higher the difference between Cₘₐₓ and NOAEL, the higher is the renal safety margin during the treatment and the lower is the likelihood for adverse events.

In some embodiments, the inventive method provides as renal safety margin a difference between Cₘₐₓ and NOAEL of about 100 ng/mL to about 2,500 ng/mL, about 150 ng/mL to about 2,500 ng/mL, about 200 ng/mL to about 2,500 ng/mL, about 300 ng/mL to about 2,250 ng/mL, about 400 ng/mL to about 2,250 ng/mL, about 500 ng/mL to about 2,000 ng/mL, about 600 ng/mL to about 2,000 ng/mL, about 600 ng/mL to about 1,800 ng/mL, about 700 ng/mL to about 1,800 ng/mL, about 800 ng/mL to about 1,800 ng/mL.

In some embodiments, the inventive method results an elimination half-life of neridronic acid in the subject, preferably a human being, of about 10 hours to about 75 hours, of about 15 hours to about 50 hours, of about 20 hours to about 35 hours, of about 22 hours to about 30 hours or of about 25 +/- 10 hours, preferably 25 +/- 5 hours, more preferably of about 25 +/- 2.5 hours, more preferably of about 24 hours, about 25 hours, about 26 hours or about 27 hours.

As used herein, the "elimination half-life" refers to the apparent first-order terminal plasma elimination half-life, obtained by non-compartmental analysis. A terminal plasma elimination half-life is the time required to reduce the plasma concentration to half after reaching pseudo-equilibrium, and not the time required to eliminate half the administered dose.

In some embodiments, the inventive method results in a desired range for an area under the plasma concentration curve (AUC) of neridronic acid in that particular subject, particularly a human being. In some embodiments, the inventive method may result in an AUC of neridronic acid of about 1,000 ng·hr/mL to about 150,000 ng·hr/mL, about 2,500 ng·hr/mL to about 125,000 ng·hr/mL, about 5,000 ng·hr/mL to about 125,000 ng·hr/mL, about 7,500 ng·hr/mL to about 100,000 ng·hr/mL, about 10,000 ng·hr/mL to about 100,000 ng·hr/mL, about 15,000 ng·hr/mL to about 90,000 ng·hr/mL, about 25,000 ng·hr/mL to about 75,000 ng·hr/mL, about 30,000 ng·hr/mL to about 65,000 ng·hr/mL, about 40,000 ng·hr/mL to about 6,000 ng·hr/mL, about 45,000 ng·hr/mL to about 55,000 ng·hr/mL, or any AUC in a range bounded by, or between, any of these values, upon administration of neridronic acid to the subject.

In some embodiments, the inventive method provides a lower Cₘₐₓ and substantially equivalent AUC of neridronic acid compared to the intravenous administration in one, two, or more individual unit doses of neridronic acid over a duration of less than 4 hours.

Unless otherwise indicated, the AUC refers to the AUC extrapolated to infinity (AUC(0-inf)).

Acute phase reactions (acute renal damage) during intravenous administration of bisphosphonates, such as neridronic acid, typically correlate with the dose and hence with Cₘₐₓ (Adami S et al, Calcif Tissue Int (1987) 41:326-331). One possibility to manage acute phase reactions during intravenous administration of bisphosphonates is the co-administration of APAP (Acetaminophen, N-(4-Hydroxy-phenyl)acetamid). High dosages of APAP, however, may also contribute to hepatic damages (Tittarelli R et al. Eur Rev Med Pharmacol Sci (2017) 21(1 Suppl):95-101).

In another embodiment of the present invention, the inventive method results in a reduced necessity to co-administer APAP.

Preferably, no co-administration of APAP is required to prevent acute phase reactions during the treatment.

The amount of neridronic acid in a dosage form (therapeutic composition) may vary. In some embodiments, liquid dosage forms for intravenous administration according to the invention may comprise about 0.0001% (w/v) to about 50% (w/v), about 0.01% (w/v) to about 20% (w/v), about 0.01% to about 25% (w/v), about 0.1% (w/v) to about 25% (w/v), about 1% (w/v) to about 15% (w/v), about 3% (w/v) to about 15% (w/v), about 5% (w/v) to about 15% (w/v), about 7% (w/v) to about 15% (w/v), about 10% (w/v) to about 15% (w/v), about 12% (w/v) to about 20% (w/v), about 20% (w/v) to about 30% (w/v), about 30% (w/v) to about 40% (w/v), or about 40% (w/v) to about 50% (w/v) of neridronic acid.

In some embodiments, the liquid dosage forms for intravenous administration may contain about 0.005 mg to about 500 mg, about 0.1 mg to about 400 mg, about 0.5 mg to about 300 mg, about 1 mg to about 500 mg, about 1 mg to about 400 mg, about 1 mg to about 300 mg, about 10 mg to about 500 mg, about 10 mg to about 400 mg, about 10 mg to about 300 mg, about 25 mg to about 500 mg, about 25 mg to about 400 mg, about 25 mg to about 300 mg, about 50 mg to about 500 mg, about 50 mg to about 400 mg, or about 50 mg to about 300 mg of neridronic acid.

In some embodiments, the liquid dosage forms for intravenous administration may contain about 150 +/- 100 mg, about 150 +/- 75 mg, about 150 +/- 50 mg, about 120 +/- 100 mg, about 120 +/- 60 mg, about 120 +/- 40 mg, about 120 +/- 30 mg, about 100 +/- 75 mg, about 100 +/- 60 mg, about 100 +/- 50 mg, about 100 +/- 30 mg, about 100 +/-25 mg, about 100 +/- 20 mg, about 100 +/- 15 mg, about 100 +/- 10 mg, about 80 +/- 50 mg, about 80 +/- 40 mg, about 80 +/- 30 mg, about 80 +/- 25 mg, about 80 +/- 20 mg, about 80 +/- 15 mg, about 80 +/- 10 mg, about 60 +/- 50 mg, about 60 +/- 40 mg, about 60 +/- 30 mg, about 60 +/- 25 mg, about 60 +/- 20 mg, about 60 +/- 15 mg, or about 60 +/- 10 mg of neridronic acid.

Neridronic acid may be combined with a pharmaceutical carrier to form the inventive dosage form. In some embodiments, the dosage form may either comprise neridronic acid as individual therapeutic agent or in a combination with other therapeutically active agents.

One embodiment of the first aspect of the invention relates to a composition comprising neridronic acid for use in a method for the treatment of complex regional pain syndrome (CRPS) or for use in a method for the treatment of pain associated with CRPS, characterized in that
an effective amount of neridronic acid is administered intravenously in one or in two or more individual unit doses, and
each dose of neridronic acid is administered to the subject by intravenous infusion over a duration of at least 3 hours, at least 4 hours or at least 5 hours.

Preferably, the inventive dosage form is an aqueous solution.

In some embodiments, the dosage form for intravenous administration comprises besides neridronic acid further excipients, such as tonicity modifiers (e.g., sodium chloride), pH-buffer agents (e.g., citric acid, sodium citrates), pH-adjusting agents (e.g., hydrochloric acid or sodium hydroxide), co-solvents or solubilizers (e.g., dimethylacetamide), alcoholic solvents (e.g., ethanol, polyethylene glycol (PEG-300, PEG-400, PEG-600, PEG-1000), propylene glycol), surfactants (e.g., polysorbate 20, polysorbate 80, lecithine), stabilizers and antioxidants (e.g., glycin, citric acid, tocopherol, butylated hydroxy-anisol (BHA), butylated hydroxytoluene (BHT), ascorbic acid), bulking agents, viscosity enhancers or viscosity reducers, chelating agents (e.g., EDTA), preservatives, and adjuvants.

A preferred dosage form for intravenous administration may comprise

| **Ingredients** | **Amounts per dosage form [mg**] |
|---|---|
| Neridronic acid | 100 |
| Sodium chloride | 1 to 50 |
| Sodium citrate dihydrate | 5 to 150 |
| Citric acid monohydrate | 5 to 100 |
| pH adjusting agents (sodium hydroxide, hydrochloric acid) | q.s. |
| Water for injection | Ad 8 mL |

A preferred embodiment of the dosage form is the dosage form available under the tradename Nerixia^{™} in Italy.

In some embodiments, the dosage form for intravenous administration has a pH-value (measured at 25°C) of about pH 4 to about pH 8, preferably of about pH 4.5 to about pH 7 and more preferably of about pH 4.5 to about pH 5.5.

In some embodiments, the dosage form for intravenous administration may be stored in vials, IV bags, ampoules, cartridges, and prefilled syringes.

In one embodiment of the present invention, a method of treating pain associated with CRPS, relief of pain can be short-term, e.g., for a period of hours after administration of the dosage form, and/or relief of pain can be long-term, e.g., lasting for days, weeks, or even months after administration of neridronic acid.

In some embodiments, a subject, such as a human being, experiences significant pain relief from pain associated with CRPS at least about 3 hours, at least about 6 hours, at least about 12 hours, at least about 24 hours, at least about 48 hours, at least about one week, at least about 2 weeks, or at least about 3 weeks after administration of neridronic acid.

In some embodiments, a mammal, such as a human being, experiences significant pain relief from pain associated with CRPS during at least part of the time from about 3 hours to about 2 weeks, about 3 hours to about 3 weeks, about 3 hours to about 24 hours, about 6 hours to about 2 weeks, or about 6 hours to about 24 hours, about 3 days to about 2 weeks, about 6 days to about 2 weeks, after administration of neridronic acid.

In some embodiments, a human being treated has significant pain relief at 3 months, 6 months, 9 months, or one year after administration of neridronic acid.

In some embodiments, a human being treated experiences prolonged pain relief from pain associated with CRPS. The terms "prolonged pain relief" as used herein are defined as relief from pain associated with CRPS for a duration of more than one day, for a duration of more than 2 days, for a duration of more than 3 days, for a duration of more than 4 days, for a duration of more than 5 days, for a duration of more than 6 days, for a duration of more than one week, for a duration of more than 2 weeks, for a duration of more than 3 weeks, for a duration of more than 4 weeks, for a duration of more than 6 weeks, for a duration of more than one month, a duration of more than 2 months, a duration of more than 3 months, a duration of more than 4 months, a duration of more than 5 months, a duration of more than 6 months, a duration of more than 9 months and preferably for a duration of more than 12 months.

In some embodiments, "significant pain relief" refers to pain relief according to the Pain Intensity Score (NRS) by at least 1, at least 2, at least 3, at least 4 points, at least 5 points, at least 6 points or at least 7 points on the NRS scale.

In some embodiments, "significant pain relief" refers to a pain relief of about at least 20 %, at least 25 %, at least 30 %, at least 35 %, at least 40 %, at least 45 %, at least 50 %, at least 60 %, at least 70 %, at least 80 % or at least 90 % in the pain intensity from baseline pain.

In some embodiments, a subject, such as a human being, experiences pain relief and thus a change from baseline to week 12 in the average pain intensity score (weekly average of pain values recorded daily in the electronic diary) by at least 1, at least 2, at least 3, or at least 4 points on the NRS scale. The Pain Intensity Score is defined as the mean value of current pain intensity ratings using an 11-point numerical rating scale (NRS) where 0 = "no pain" and 10 = "pain as bad as you can imagine".

In some embodiments, the subject, such as a human being, experiences a change from baseline to week 12 in the average pain intensity score by at least 1, at least 2, at least 3, at least 4 points, at least 5 points, at least 6 points or at least 7 points on the NRS scale or at least 20 %, at least 25 %, at least 30 %, at least 35 %, at least 40 %, at least 45 %, at least 50 %, at least 60 %, at least 70 %, at least 80 % or at least 90 % (as recorded by the subject).

The Average Pain Intensity Score is defined as the mean value of current pain intensity ratings. Subjects will rate their average pain intensity once daily using an 11-point numerical rating scale where 0 = "no pain" and 10 = "pain as bad as you can imagine". Average pain intensity ratings are in reference to the CRPS-affected limb.

In some embodiments, the proportion of subjects, such as a human being, experiencing a change from baseline to week 12 in average pain intensity score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, a subject, such as a human being, experiences pain relief and thus a change from baseline to week 26 in the average pain intensity score by at least 1, at least 2, at least 3, at least 4 points, at least 5 points, at least 6 points or at least 7 points on the NRS scale.

In some embodiments, the subject, such as a human being, experiences a change from baseline to week 26 in the average pain intensity score by at least 20 %, at least 25 %, at least 30 %, at least 35 %, at least 40 %, at least 45 %, at least 50 %, at least 60 %, at least 70 %, at least 80 % or at least 90 % (as recorded by the subject).

In some embodiments, the proportion of subjects, such as a human being, experiencing pain relief and thus a change from baseline to week 26 according to the average pain intensity score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, a subject, such as a human being, experiences pain relief and thus a change from baseline to week 52 in the average pain intensity score (weekly average of pain values recorded daily in the electronic diary) (NRS) by at least 1, at least 2, at least 3, at least 4 points, at least 5 points, at least 6 points or at least 7 points on the NRS scale.

In some embodiments, the subject, such as a human being, experiences a change from baseline to week 52 in the average pain intensity score by at least 20 %, at least 25 %, at least 30 %, at least 35 %, at least 40 %, at least 45 %, at least 50 %, at least 60 %, at least 70 %, at least 80 % or at least 90 % (as recorded by the subject).

In some embodiments, the proportion of subjects, such as a human being, experiencing pain relief and thus a change from baseline to week 52 according to the average pain intensity score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, a subject, such as a human being, experiences pain relief and thus a change from week 26 to week 52 in the average pain intensity score by at least 1, at least 2, at least 3, at least 4 points, at least 5 points, at least 6 points or at least 7 points on the NRS scale.

In some embodiments, the subject, such as a human being, experiences a change from week 26 to week 52 in the average pain intensity score by at least 20 %, at least 25 %, at least 30 %, at least 35 %, at least 40 %, at least 45 %, at least 50 %, at least 60 %, at least 70 %, at least 80 % or at least 90 % (as recorded by the subject).

In some embodiments, the proportion of subjects, such as a human being, experiencing pain relief and thus a change from week 26 to week 52 according to the average pain intensity score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, a subject, such as a human being, experiences pain relief in the dynamic mechanical allodynia (DMA) and thus a change from baseline to week 12, in the pain intensity level of DMA.

Allodynia is defined as pain in response to a non-nociceptive stimulus. In cases of mechanical allodynia, even gentle mechanical stimuli such as a slight bending of hairs can evoke pain. In investigating DMA, a tactile stimulus is applied in a single sweeping motion (1 cm to 2 cm length) on the skin on the affected limb. The subjects are asked to judge the stimulus intensity by means of an NRS (0 to 10). "0" in this case means "no pain". Each "pricking", "stinging" or "burning" sensation is defined as a painful sensation, which should always be evaluated by giving a value greater than "0". A value of "10" corresponds to the individual maximum pain imaginable.

In some embodiments, the subject, such as a human being, experiences pain relief and thus a change from baseline to week 12 in the pain intensity level of DMA by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing pain relief and thus a change from baseline to week 12 in the pain intensity level of DMA by at least 1, at least 2, at least 3, at least 4 points, at least 5 points, at least 6 points or at least 7 points on the NRS scale or by at least 20 %, at least 25 %, at least 30 %, or at least 40 % from baseline in the average pain intensity at week 12, is at least 30%, at least 40%, at least 50%, at least 60%, at least 70%, at least 80%, at least 90% of the total number of subjects treated.

In some embodiments, a subject, such as a human being, experiences pain relief in the dynamic mechanical allodynia (DMA) and thus a change from baseline to week 6, week 26, week 36, or week 52 in the pain intensity level of DMA.

In some embodiments, the subject, such as a human being, experiences pain relief and thus a change from baseline to week 6, week 26, week 36, or week 52 in the pain intensity level of DMA by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing pain relief and thus a change from baseline to week 6, week 26, week 36, or week 52 in the pain intensity level of DMA by at least 1, at least 2, at least 3, at least 4 points, at least 5 points, at least 6 points or at least 7 points on the NRS scale or by at least 20 %, at least 25 %, at least 30 %, or at least 40 % from baseline in the average pain intensity at week 12, is at least 30%, at least 40%, at least 50%, at least 60%, at least 70%, at least 80%, at least 90% of the total number of subjects treated.

In some embodiments, a subject, such as a human being, experiences change from baseline to week 12 in the pressure pain threshold (PPT) ratio.

The PPT is assessing the level of pressure causing deep joint or muscle pain. In CRPS subjects, light, normally non-painful pressure applied at joints or muscles causes pain in the deep somatic tissues.

In investigating the Pressure pain threshold using a pressure algometer (contact area 1 cm²), the threshold for pressure-induced pain is measured on the thenar muscle / abductor hallucis muscle in 3 series of slowly increasing stimulus intensities (at a rate of about 50 kPa/s). The threshold is then determined as the arithmetic mean of the 3 series (in kPa).

The ratio of the thresholds of the affected limb versus the unaffected limb will be calculated.

In some embodiments, the subject, such as a human being, experiences a change from baseline to week 12 in the PPT ratio for the thenar muscle/abductor hallucis muscle, preferably by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from baseline to week 12 in the PPT ratio for the thenar muscle/abductor hallucis muscle, preferably by at least 20 %, at least 25 %, at least 30 %, or at least 40 % from baseline, is at least 30%, at least 40%, at least 50%, at least 60%, at least 70%, at least 80%, at least 90% of the total number of subjects treated.

In some embodiments, a subject, such as a human being, experiences a change from baseline to week 6, week 26, week 36, or week 52 in the pressure pain threshold (PPT) ratio.

In some embodiments, the subject, such as a human being, experiences a change from baseline to week 6, week 26, week 36, or week 52 in the PPT ratio for the thenar muscle/abductor hallucis muscle, preferably by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from baseline to week 6, week 26, week 36, or week 52 in the PPT ratio for the thenar muscle/abductor hallucis muscle, preferably by at least 20 %, at least 25 %, at least 30 %, or at least 40 % from baseline, is at least 30%, at least 40%, at least 50%, at least 60%, at least 70%, at least 80%, at least 90% of the total number of subjects treated.

In some embodiments, a subject, such as a human being, experiences a change, in particular a reduction, on edema of the hand or the foot at week 12 after the start of the treatment.

To investigate the change on edema, in subjects with the CRPS sign of edema on the CRPS Severity Score at baseline, circumference of the hand or foot will be measured by the investigator with measurement tape using the figure-of-eight method at both the affected limb and the contralateral unaffected limb. Preferably, an average of the 3 measurements will be used for further analysis. The ratio of the averages of the affected limb versus the unaffected limb will be calculated.

In some embodiments, the subject, such as a human being, experiences a reduction from baseline to week 12 in the ratio of the figure of eight measurements of the affected limb versus the unaffected limb, preferably by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from baseline to week 12 in the ratio of the figure of eight measurements of the affected limb versus the unaffected limb, preferably by at least 20 %, at least 25 %, at least 30 %, or at least 40 % from baseline, is at least 30%, at least 40%, at least 50%, at least 60%, at least 70%, at least 80%, at least 90% of the total number of subjects treated.

In some embodiments, a subject, such as a human being, experiences a change, from baseline to week 6, week 26, week 36, or week 52, in the ratio of the figure of eight measurements of the affected limb versus the unaffected limb.

In some embodiments, the subject, such as a human being, experiences a reduction from baseline to week 6, week 26, week 36, or week 52 in the ratio of the figure of eight measurements of the affected limb versus the unaffected limb, preferably by at least 20 %, at least 25 %, at least 30 %, at least 35 %, at least 40 %, at least 45 %, at least 50 %, at least 60 %, at least 70 %, at least 80 % or at least 90 %.

In some embodiments, the subject, such as a human being, exhibits the signs and symptoms of CRPS in an affected limb (e.g., arm or leg) and exhibits asymmetry with respect to the contralateral limb. In other embodiments, the subject, such as a human being, has a fracture as a precipitating event to the signs and symptoms of CRPS.

In some embodiments, the proportion of subjects, such as a human being, experiencing a change from baseline week 6, week 26, week 36, or week 52 in the ratio of the figure of eight measurements of the affected limb versus the unaffected limb, preferably by at least 20 %, at least 25 %, at least 30 %, or at least 40 % from baseline, is at least 30%, at least 40%, at least 50%, at least 60%, at least 70%, at least 80%, at least 90% of the total number of subjects treated.

In some embodiments, the subject, such as a human being, experiences improvement according to the Patient Global Impression of Change (PGIC) from baseline to week 6, week 12, week 16, week 16, week 22, week 26, week 28, week 36 and week 52 after the start of the treatment by at least 1, at least 2, at least 3 or at least 4 ratings.

The PGIC is defined as a self-reported measure of perceived change in overall condition since the start of the study. Subjects will select one of seven responses ranging from very much improved to very much worse. A response of very much improved or much improved is generally regarded as a clinically important outcome.

In some embodiments, the proportion of subjects, such as a human being, experiencing improvement according to the Patient Global Impression of Change (PGIC) from baseline to week 6, week 12, week 16, week 16, week 22, week 26, week 28, week 36 and week 52 after the start of the treatment by at least 1, at least 2, at least 3 or at least 4 ratings, is at least 30%, at least 40%, at least 50%, at least 60%, at least 70%, at least 80%, at least 90% of the total number of subjects treated.

In some embodiments, the subject, such as a human being, experiences improvement according to the Patient Global Impression of Severity (PGI-S) from baseline to week 6, week 12, week 16, week 16, week 22, week 26, week 28, week 36 and week 52 after the start of the treatment, preferably by at least 1, at least 2, at least 3 or at least 4 ratings.

The PGI-S is a global index that may be used to rate the severity of a specific condition (a single state scale). Typically, it is a 1-item questionnaire designed to assess a patient's impression of disease severity. Subjects are asked to best describe their CRPS symptoms over the past week with 1 of 5 possible responses (none, mild, moderate, severe, very severe). It is a simple, direct, easy-to-use scale that is intuitively understandable to clinicians

In some embodiments, the proportion of subjects, such as a human being, experiencing improvement according to the Patient Global Impression of Severity (PGI-S) from baseline to week 6, week 12, week 16, week 16, week 22, week 26, week 28, week 36 and week 52 after the start of the treatment, preferably by at least 1, at least 2, at least 3 or at least 4 ratings, is at least 30%, at least 40%, at least 50%, at least 60%, at least 70%, at least 80%, at least 90% of the total number of subjects treated.

In some embodiments, the proportion of subjects, such as a human being, experiencing a change from week 26 to week 52 according to the PGI-S, preferably by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the proportion of subjects, such as a human being, experiencing a change from week 26 to week 52 according to the PGI-S, preferably by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change from baseline to week 12 and/or to week 26 and/or to week 52 after the start of the treatment according to the EuroQol-5 Dimension 5 Level (EQ-5D-5L) Index Score, preferably by at least 20 %, at least 25 %, at least 30 %, at least 35 %, at least 40 %, at least 45 %, at least 50 %, at least 60 %, at least 70 %, at least 80 % or at least 90 %.

The EQ-5D-5L Index Score is defined as description of the subject's overall health status and is derived from self-reported scores in 5 health dimensions: mobility, self-care, usual activities, pain and discomfort, and anxiety and depression. Subjects will rate each dimension at one of 5 levels, with level 1 indicating the best health state (no problems) and level 5 indicating worst health state (e.g., unable to walk about). The EQ-5D-5L Index Score ranges from 0 to 1, with 0 representing death and 1.0 representing perfect health.

In some embodiments, the proportion of subjects, such as a human being, experiencing a change from baseline to week 12 and/or to week 26 and/or to week 52 after the start of the treatment in the EQ-5D-5L Index Score by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change from week 26 to week 52 according to the EuroQol-5 Dimension 5 Level (EQ-5D-5L) Index Score, preferably by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from week 26 to week 52 according to the EuroQol-5 Dimension 5 Level (EQ-5D-5L) Index Score, preferably by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change from baseline according to the health-related visual analog scale (VAS) score to week 12 and/or to week 26 and/or to week 52 after the start of the treatment, preferably by at least 20 %, at least 25 %, at least 30 %, at least 35 %, at least 40 %, at least 45 %, at least 50 %, at least 60 %, at least 70 %, at least 80 % or at least 90 %.

The health-related visual analog scale (VAS) score is defined as a self-reported measure of the subject's overall health "today". Subjects will place a mark on a 20 cm vertical scale numbered from 0 to 100, with 0 labeled as "the worst health you can imagine" and 100 labeled as "the best health you can imagine". The EQ VAS ranges from 0 to 100, with higher scores representing better overall health.

In some embodiments, the proportion of subjects, such as a human being, experiencing a change from baseline in the VAS Score to week 12 and/or to week 26 and/or to week 52 after the start of the treatment by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change from week 26 to week 52 according to the VAS Score, preferably by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from week 26 to week 52 according to the VAS Score, preferably by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change from baseline to week 6, week 12, week 16, week 16, week 22, week 26, week 28, week 36 and week 52 in the active range of motion (AROM) ratio (affected limb and unaffected limb) measured in the hand or foot, respectively, preferably by at least 20 %, at least 25 %, at least 30 %, at least 35 %, at least 40 %, at least 45 %, at least 50 %, at least 60 %, at least 70 %, at least 80 % or at least 90 %.

The AROM is measured using a universal hand-held goniometer, based on the guidance recommended by the American Medical Association guidelines and by the American Society of Hand Therapists' clinical assessment recommendations.

The AROM is defined as the range of movement through which a subject can actively (without assistance) move a joint using the adjacent muscles. Measuring the AROM does not involve any external stimulation; the subject is not touched and decides how far to move. The magnitude of the AROM can be influenced by other factors not directly related to the joint, such as the subject's motivation. In order to maximize the reliability of measurements, a rigid measurement protocol and valid and reliable measurement instruments were developed. Detailed instructions and in-depth training will be provided to the investigators. The subject has to be instructed not to exceed the pain limit in order to avoid increase of complaints, which could influence future measurements and treatment outcome.

For subjects having decreased AROM at baseline (diagnosed based on clinical judgment; sign "Motor abnormalities" ticked "yes", sub-category "decreased AROM" ticked "yes" on the CRPS Severity Score at baseline), both affected and unaffected limbs will be measured using a goniometer, first the unaffected extremity to accustom the patient to the measurements, followed by the affected extremity. The AROM is measured only during active flexion and extension (i.e., sagittal plane, transversal axis) of the wrist or ankle, respectively. The ratio of the AROM of the affected and the unaffected limb will be calculated.

In some embodiments, the proportion of subjects, such as a human being, experiencing a change from baseline to week 6, week 12, week 16, week 16, week 22, week 26, week 28, week 36 and week 52 in the active range of motion (AROM) ratio (affected limb and unaffected limb) measured in the hand or foot, respectively, preferably by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change according to the Worst Pain Intensity Score from baseline to week 12 by at least 1, at least 2, at least 3, at least 4 points, at least 5 points, at least 6 points or at least 7 points on the NRS scale.

The Worst Pain Intensity Score is defined as the mean value of worst pain intensity ratings. Subjects will rate their worst pain intensity once daily using an 11-point numerical rating scale where 0 = "no pain" and 10 = "pain as bad as you can imagine". Worst pain intensity ratings are in reference to the CRPS-affected limb.

In some embodiments, the subject, such as a human being, experiences a change from baseline to week 12 in the Worst Pain Intensity Score by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from baseline to 12 in the Worst Pain Intensity Score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change according to the Worst Pain Intensity Score from baseline to week 26 or week 52 by at least 1, at least 2, at least 3, at least 4 points, at least 5 points, at least 6 points or at least 7 points on the NRS scale.

In some embodiments, the subject, such as a human being, experiences a change from baseline to week 26 or week 52 in the Worst Pain Intensity Score by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from baseline to week 26 or week 52 in the Worst Pain Intensity Score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change from week 26 to week 52 in the Worst Pain Intensity Score by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from week 26 to week 52 in the Worst Pain Intensity Score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change according to the Current Pain Intensity Score from baseline to week 12 by at least 1, at least 2, at least 3, at least 4 points, at least 5 points, at least 6 points or at least 7 points on the NRS scale.

The Current Pain Intensity Score is defined as the mean value of current pain intensity ratings. Subjects will rate their current pain intensity once daily using an 11-point numerical rating scale where 0 = "no pain" and 10 = "pain as bad as you can imagine". Current pain intensity ratings are in reference to the CRPS-affected limb.

In some embodiments, the subject, such as a human being, experiences a change from baseline to week 12 in the Current Pain Intensity Score by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from baseline to week 12 in the Current Pain Intensity Score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change according to the Current Pain Intensity Score from baseline to week 26 or week 52 by at least 1, at least 2, at least 3, at least 4 points, at least 5 points, at least 6 points or at least 7 points on the NRS scale.

In some embodiments, the subject, such as a human being, experiences a change from baseline to week 26 or week 52 in the Current Pain Intensity Score by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from baseline to week 26 or week 52 in the Current Pain Intensity Score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments, the subject, such as a human being, experiences a change according to the Current Pain Intensity Score from week 26 to week 52 by at least 1, at least 2, at least 3, at least 4 points, at least 5 points, at least 6 points or at least 7 points on the NRS scale.

In some embodiments, the subject, such as a human being, experiences a change from week 26 to week 52 in the Current Pain Intensity Score by at least 20 %, at least 25 %, at least 30 %, at least 35 %, at least 40 %, at least 45 %, at least 50 %, at least 60 %, at least 70 %, at least 80 % or at least 90 %.

In some embodiments, the proportion of subjects, such as a human being, experiencing a change from week 26 to week 52 in the Current Pain Intensity Score by at least 1, at least 2, at least 3, at least 4 points, at least 5 points, at least 6 points or at least 7 points on the NRS scale or by at least 20 %, at least 25 %, at least 30 %, at least 35 %, at least 40 %, at least 45 %, at least 50 %, at least 60 %, at least 70 %, at least 80 % or at least 90 %, is at least 30%, at least 40%, at least 50%, at least 60%, at least 70%, at least 80%, at least 90% of the total number of subjects treated.

In some embodiments of the first aspect of the invention, a subject being treated for CRPS or pain associated with CRPS exhibits the sign and symptoms of CRPS for a duration of at least 1 month, for at least 2 months, for at least 6 months, or for at least 1 year.

According to the invention, the duration a subject exhibits the sign and symptoms of CRPS is defined as the duration since the onset of the signs and symptoms of CRPS. The signs and symptoms of CRPS are determined according to the clinical diagnostic criteria recommended by the International Association for the Study of Pain (IASP; "Budapest clinical criteria") (Harden RN, et al. Pain Med. 2013 14 (2):180-229). Such analysis may also be performed retrospectively.

In some embodiments of the first aspect of the invention, a subject being treated for CRPS or pain associated with CRPS exhibits the sign and symptoms of CRPS for a duration of 5 years or less, 4 years or less, 3 years or less, 30 months or less, 2 years or less, 18 months or less, 12 months or less, 9 months or less, or 6 months or less.

In preferred embodiments of the first aspect of the invention, a subject, such as a human being, being treated for CRPS or pain associated with CRPS, and preferably experiencing the above mentioned pain relief, changes, improvements or reductions, exhibits the sign and symptoms of CRPS for a duration of 9 months or less, 1 year or less, 15 months or less, 18 months or less, 21 months or less, 2 years or less, 27 months or less, 30 months or less, 33 months or less, 3 years or less, 42 months or less, 4 years or less, or 5 years or less,
preferably for a duration of 9 months or less, 1 year or less, 15 months or less, 18 months or less, 21 months or less, 2 years or less, 30 months or less, 3 years or less, or 4 years or less,
more preferably for a duration of 1 year or less, 18 months or less, 2 years or less than 3 years,
and even more preferably for a duration of less than 2 years.

In a second aspect, the invention further provides a method of treatment for complex regional pain syndrome (CRPS), which comprises administering to a subject in need of CRPS treatment an effective amount of neridronic acid, that is effective for providing treatment of CRPS, wherein the effective amount of neridronic acid is administered intravenously in one or as two or more individual unit doses, characterized in that the subject exhibits the sign and symptoms of CRPS for a duration of 5 years or less.

In one embodiment of the second aspect, the invention further provides neridronic acid for use in a method for the treatment of complex regional pain syndrome (CRPS) or for use in a method for the treatment of pain associated with CRPS, wherein a therapeutically effective amount of neridronic acid is administered intravenously in one or as two or more individual unit doses, and characterized in that the subject exhibits the sign and symptoms of CRPS for a duration of 5 years or less.

In some embodiments of the second aspect of the invention, the method of treatment for CRPS relates to the treatment for pain associated with CRPS.

In preferred embodiments of the second aspect of the invention, a subject, such as a human being, being treated for CRPS or pain associated with CRPS by administration of neridronic acid, and preferably experiencing the above mentioned pain relief, changes, improvements or reductions, exhibits the sign and symptoms of CRPS for a duration of 9 months or less, 1 year or less, 15 months or less, 18 months or less, 21 months or less, 2 years or less, 27 months or less, 30 months or less, 33 months or less, 3 years or less, 42 months or less, 4 years, or 5 years or less,
preferably for a duration of 9 months or less, 1 year or less, 15 months or less, 18 months or less, 21 months or less, 2 years or less, 30 months or less, 3 years, or 4 years or less, more preferably for a duration of 1 year or less, 18 months or less, 2 years or less, or 3 years or less,
and even more preferably for a duration of 2 years or less.

The embodiments of the other aspects of the invention apply analogously to the second aspect of the invention and vice versa.

In a third aspect, the invention further provides a kit comprising at least one container comprising, consisting essentially of, or consisting of a therapeutically effective amount of neridronic acid; and optionally instructions for administration of the container(s). For example, the invention provides a kit comprising: a first container comprising, consisting essentially of, or consisting of a therapeutically effective amount of neridronic acid; or a first container and a second container, both of which comprise, consist essentially of, or consist of a therapeutically effective amount of neridronic acid; or a first container, a second container, and a third container, each of which comprise, consist essentially of, or consist of a therapeutically effective amount of neridronic acid; or a first container, a second container, a third container, and a fourth container, each of which comprise, consist essentially of, or consist of a therapeutically effective amount of neridronic acid; and so on. The total amount of neridronic acid that may be present in the one or more containers is about 100 mg to about 750 mg, such as, for example, about 150 mg, or about 200 mg, or about 250 mg, or about 300 mg, or about 400 mg, or about 500 mg. In other words, if the kit contains four containers, for example, and a total amount of about 100 mg of neridronic acid is to be administered, each of the four containers may contain about 25 mg of neridronic acid.

The one or more containers of the kit may allow for about 1 mL to about 20 mL of total contents, such as about 5 mL to about 10 mL, preferably about 8 mL. And the total contents of the one or more containers may also be diluted in saline, for example, in order to achieve a desired total dose of neridronic acid. For example, a first container (8 mL) may contain about 100 mg of neridronic acid, and the entire first container may be diluted in 500 mL saline and administered to the subject in need thereof, resulting in a total administered dose of about 100 mg of neridronic acid. Alternatively, for example, 6 mL of a first container (8 mL, 100 mg neridronic acid) may be diluted in 500 mL saline and administered to the subject in need thereof, resulting in a total administered dose of about 75 mg of neridronic acid. Or, for example, 5 mL of a first container (8 mL, 100 mg neridronic acid) may be diluted in 500 mL saline and administered to the subject in need thereof, resulting in a total administered dose of about 62.5 mg of neridronic acid.

In this way, the one or more containers may be administered to a subject according to any one of the methods of the invention. For example, a kit of the invention may contain four 8 mL containers, each of which contains 100 mg of neridronic acid, and the entire contents of each of the four containers may be administered to the subject on day 1, day 3, day 5, and day 7, or on day 1, day 4, day 7, and day 10, or on day 1, day 5, day 9, and day 13, and the contents of each of the four containers may be administered intravenously to the subject over a duration of at least 4 hours. In order to provide a total administered dose equal to about 400 mg of neridronic acid to the subject, for example, the total contents of the first container (i.e., 8 mL) may be diluted in 500 mL of saline on day 1, and administered intravenously to the subject over a duration of at least 4 hours, then the total contents of the second container may be diluted in 500 mL of saline on day 4, and administered intravenously to the subject over a duration of at least 4 hours, then the total contents of the third container may be diluted in 500 mL of saline on day 7, and administered intravenously to the subject over a duration of at least 4 hours, and finally the total contents of the fourth container may be diluted in 500 mL of saline on day 10, and administered intravenously to the subject over a duration of at least 4 hours. Using the same kit having four containers, in order to provide a total administered dose equal to about 300 mg of neridronic acid to the subject, for example, 6 mL of each of the 8 mL containers containing about 100 mg of neridronic acid may be diluted in 500 mL of saline on successive administration days 1, 4, 7, and 10, for example, and the diluted contents of each container may be administered intravenously to the subject over a duration of at least 4 hours on each successive administration day. And using the same kit having four containers, in order to provide a total administered dose equal to about 250 mg of neridronic acid to the subject, for example, 5 mL of each of the 8 mL containers containing about 100 mg of neridronic acid may be diluted in 500 mL of saline on successive administration days 1, 4, 7, and 10, for example, and the diluted contents of each container may be administered intravenously to the subject over a duration of at least 4 hours on each successive administration day.

Instead of 500 mL saline, other volumes, such as 250 mL or 1000 mL, or other physiologically suitable solutions for injection (such as a dextrose solution) may be used when applying the contents of the containers.

Preferably, a kit of the invention contains a first container (8 mL) containing about 100 mg of neridronic acid. Also preferred is a kit that contains a first container (8 mL), a second container (8 mL), a third container (8 mL), and a fourth container (8 mL), and each of the four containers contains about 100 mg of neridronic acid.

The term "container" as used herein refers to any receptacle or applicator means capable of holding, storing, and/or applying the neridronic acid. Such a container may be in any container configuration known to a person skilled in the art, such as, but not limited to, a vial, an IV bag, an ampoule, a cartridge, a syringe, a pouch, a bottle, a jar, or a box. The containers may be made of any material suitable for the precursor materials contained therein and additionally suitable for short and/or long-term storage under any kind of temperature. Such materials include, by way of example, inorganic materials, such as Type I glass (including amber colored glass), ceramics, metals (e.g., aluminum, tin, and tin coated tubes), etc., and organic materials such as inert polymers including polyolefins (e.g., high density polyethylene), fluorinated polyolefins, and the like. Suitable containers include those that maintain the sterility and integrity of their contents, for example, by providing a barrier to moisture. The preferred container is also one which is compatible with any chosen method of sterilization, including, for example, irradiation. The suitable containers may have an appropriate applicator means to dispense the neridronic acid from the container to the subject. The containers may be sealed as separate articles or may be combined into a single article of manufacture having a barrier between the containers. Such barriers include frangible or crushable barriers or envelopes. In a preferred embodiment of the invention, the container is a vial.

As used herein, the term "instructions" when used in the context of a kit includes a publication, a recording, a diagram, or any other medium of expression which can be used to communicate the usefulness of the kit for its designated use. The instructions can, for example, be affixed to or included within a container for the kit.

As discussed above, the kit of the invention may be used for in any of the aforementioned methods of treating CRPS or pain associated with CRPS.

The embodiments of the other aspects of the invention apply analogously to the third aspect of invention and vice versa.

In a fourth aspect, the invention further provides the use of neridronic acid for the preparation of a pharmaceutical composition for the treatment of complex regional pain syndrome (CRPS) or pain associated with CRPS, wherein a subject in need is treated with an effective amount of neridronic acid, wherein the effective amount of neridronic acid is administered intravenously in one or as two or more individual unit doses, wherein each dose of neridronic acid is administered to the subject by intravenous infusion over a duration of at least 3 hours, at least 4 hours or at least 5 hours.

The embodiments of the other aspects of the invention apply analogously to the fourth aspect of invention and vice versa.

In a fifth aspect, the invention further provides a method of treating complex regional pain syndrome (CRPS) or pain associated with CRPS, comprising administering to a subject in need thereof a therapeutically effective amount of neridronic acid, the method comprising the steps of:
(a) determining whether the subject has reduced or impaired renal function by:
   obtaining or having obtained a biological sample from the subject; and
   determining or having determined an estimated glomerular filtrate rate (eGFR) on the biological sample using the 2009 Chronic Kidney Disease Epidemiology Collaboration creatinine equation;
(b)
   i. if the subject has an eGFR of 60 mL/min/1.73 m² or greater, then administering intravenously about 400 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 400 mg of neridronic acid, and administering each individual unit dose by intravenous infusion over a duration of at least 4 hours;
   ii. if the subject has an eGFR of 45 to less than 60 mL/min/1.73 m², then administering intravenously about 300 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 300 mg of neridronic acid, and administering each individual unit dose by intravenous infusion over a duration of at least 4 hours;
   iii. if the subject has an eGFR of 30 to less than 45 mL/min/1.73 m², then administering intravenously about 250 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 250 mg of neridronic acid, and administering each individual unit dose by intravenous infusion over a duration of at least 4 hours;
      wherein the renal safety margin for acute renal damage in the subject having an eGFR of 60 mL/min/1.73 m² or greater is higher than it would be if the 400 mg of neridronic acid was administered intravenously to the subject in 4 equal individual unit doses on day 1, day 3, day 5, and day 7, or on day 1, day 4, day 7, and day 10, or on day 1, day 5, day 9, and day 13, and each individual unit dose was administered by intravenous infusion over a duration of less than 4 hours;
      wherein the renal safety margin for acute renal damage in the subject having an eGFR of 45 to less than 60 mL/min/1.73 m² is higher than it would be if the 300 mg of neridronic acid was administered intravenously to the subject in 4 equal individual unit doses on day 1, day 3, day 5, and day 7, or on day 1, day 4, day 7, and day 10, or on day 1, day 5, day 9, and day 13, and each individual unit dose was administered by intravenous infusion over a duration of less than 4 hours;
      wherein the renal safety margin for acute renal damage in the subject having an eGFR of 30 to less than 45 mL/min/1.73 m² is higher than it would be if the 250 mg of neridronic acid was administered intravenously to the subject in 4 equal individual unit doses on day 1, day 3, day 5, and day 7, or on day 1, day 4, day 7, and day 10, or on day 1, day 5, day 9, and day 13, and each individual unit dose was administered by intravenous infusion over a duration of less than 4 hours.

In one embodiment of the fifth aspect, the invention further provides neridronic acid for use in a method of treating complex regional pain syndrome (CRPS) or pain associated with CRPS, comprising administering to a subject in need thereof a therapeutically effective amount of neridronic acid, the method comprising the steps of:
(a) determining whether the subject has reduced or impaired renal function by:
   obtaining or having obtained a biological sample from the subject; and
   determining or having determined an estimated glomerular filtrate rate (eGFR) on the biological sample using the 2009 Chronic Kidney Disease Epidemiology Collaboration creatinine equation;
(b)
   i. if the subject has an eGFR of 60 mL/min/1.73 m² or greater, then administering intravenously about 400 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 400 mg of neridronic acid, and administering each individual unit dose by intravenous infusion over a duration of at least 4 hours;
   ii. if the subject has an eGFR of 45 to less than 60 mL/min/1.73 m², then administering intravenously about 300 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 300 mg of neridronic acid, and administering each individual unit dose by intravenous infusion over a duration of at least 4 hours;
   iii. if the subject has an eGFR of 30 to less than 45 mL/min/1.73 m², then administering intravenously about 250 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 250 mg of neridronic acid, and administering each individual unit dose by intravenous infusion over a duration of at least 4 hours;
      wherein the renal safety margin for acute renal damage in the subject having an eGFR of 60 mL/min/1.73 m² or greater is higher than it would be if the 400 mg of neridronic acid was administered intravenously to the subject in 4 equal individual unit doses on day 1, day 3, day 5, and day 7, or on day 1, day 4, day 7, and day 10, or on day 1, day 5, day 9, and day 13, and each individual unit dose was administered by intravenous infusion over a duration of less than 4 hours;
      wherein the renal safety margin for acute renal damage in the subject having an eGFR of 45 to less than 60 mL/min/1.73 m² is higher than it would be if the 300 mg of neridronic acid was administered intravenously to the subject in 4 equal individual unit doses on day 1, day 3, day 5, and day 7, or on day 1, day 4, day 7, and day 10, or on day 1, day 5, day 9, and day 13, and each individual unit dose was administered by intravenous infusion over a duration of less than 4 hours;
      wherein the renal safety margin for acute renal damage in the subject having an eGFR of 30 to less than 45 mL/min/1.73 m² is higher than it would be if the 250 mg of neridronic acid was administered intravenously to the subject in 4 equal individual unit doses on day 1, day 3, day 5, and day 7, or on day 1, day 4, day 7, and day 10, or on day 1, day 5, day 9, and day 13, and each individual unit dose was administered by intravenous infusion over a duration of less than 4 hours.

In one embodiment of the inventive method, the subject has an eGFR of 60 mL/min/1.73 m² or greater,
wherein about 400 mg of neridronic acid is administered intravenously to the subject in 4 equal individual unit doses on day 1, day 4, day 7, and day 10, to result in a total administered dose equal to 400 mg of neridronic acid, and administering each individual unit dose by intravenous infusion over a duration of at least 4 hours, and
wherein the renal safety margin for acute renal damage in the subject having an eGFR of 60 mL/min/1.73 m² or greater is higher than it would be if the 400 mg of neridronic acid was administered intravenously to the subject in 4 equal individual unit doses on day 1, day 4, day 7, and day 10, and each individual unit dose was administered by intravenous infusion over a duration of less than 4 hours.

In one embodiment of the inventive method, the subject has an eGFR of 45 to less than 60 mL/min/1.73 m²,
wherein about 300 mg of neridronic acid is administered intravenously to the subject in 4 equal individual unit doses on day 1, day 4, day 7, and day 10, to result in a total administered dose equal to 300 mg of neridronic acid, and administering each individual unit dose by intravenous infusion over a duration of at least 4 hours, and
wherein the renal safety margin for acute renal damage in the subject having an eGFR of 45 to less than 60 mL/min/1.73 m² is higher than it would be if the 300 mg of neridronic acid was administered intravenously to the subject in 4 equal individual unit doses on day 1, day 4, day 7, and day 10, and each individual unit dose was administered by intravenous infusion over a duration of less than 4 hours.

In one embodiment of the inventive method, the subject has an eGFR of 30 to less than 45 mL/min/1.73 m²,
wherein about 250 mg of neridronic acid is administered intravenously to the subject in 4 equal individual unit doses on day 1, day 4, day 7, and day 10, to result in a total administered dose equal to 250 mg of neridronic acid, and administering each individual unit dose by intravenous infusion over a duration of at least 4 hours, and
wherein the renal safety margin for acute renal damage in the subject having an eGFR of 30 to less than 45 mL/min/1.73 m² is higher than it would be if the 250 mg of neridronic acid was administered intravenously to the subject in 4 equal individual unit doses on day 1, day 4, day 7, and day 10, and each individual unit dose was administered by intravenous infusion over a duration of less than 4 hours.

One embodiment of this aspect of the invention is a method, wherein the method provides a renal safety margin of about 1.2 as determined by the ratio of no-observed-adverse-effect level (NOAEL) over Cₘₐₓ.

Yet one embodiment of this aspect of the invention is a method, wherein the method provides renal safety margin of about 100 ng/mL to about 2,500 ng/mL as determined by the difference between Cₘₐₓ and no-observed-adverse-effect level (NOAEL).

The embodiments of the other aspects of the invention apply analogously to the fifth aspect of invention and vice versa.

In a sixth aspect, the invention further provides a method of treating complex regional pain syndrome (CRPS) or pain associated with CRPS, comprising administering to a subject in need thereof a therapeutically effective amount of neridronic acid, the method comprising the steps of:
(a) determining whether the subject exhibits the signs and symptoms of CRPS for a duration of 2 years or less, and/or exhibits the signs and symptoms of CRPS in an affected limb and exhibits asymmetry with respect to the contralateral limb;
(b) if the subject exhibits the signs and symptoms of CRPS for a duration of 2 years or less, and/or exhibits the signs and symptoms of CRPS in an affected limb and exhibits asymmetry with respect to the contralateral limb, then administering intravenously a therapeutically effective amount of about 250 mg, or about 300 mg, or about 400 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to the therapeutically effective amount of neridronic acid, and administering each individual unit dose by intravenous infusion over a duration of at least 4 hours.

In one embodiment of the sixth aspect, the invention further provides neridronic acid for use in a method of treating complex regional pain syndrome (CRPS) or pain associated with CRPS, comprising administering to a subject in need thereof a therapeutically effective amount of neridronic acid, the method comprising the steps of:
(a) determining whether the subject exhibits the signs and symptoms of CRPS for a duration of 2 years or less, and/or exhibits the signs and symptoms of CRPS in an affected limb and exhibits asymmetry with respect to the contralateral limb;
(b) if the subject exhibits the signs and symptoms of CRPS for a duration of 2 years or less, and/or exhibits the signs and symptoms of CRPS in an affected limb and exhibits asymmetry with respect to the contralateral limb, then administering intravenously a therapeutically effective amount of about 250 mg, or about 300 mg, or about 400 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to the therapeutically effective amount of neridronic acid, and administering each individual unit dose by intravenous infusion over a duration of at least 4 hours.

One embodiment of this aspect of the invention is a method, wherein about 250 mg is administered intravenously to the subject in 4 equal individual unit doses on day 1, day 4, day 7, and day 10, to result in a total administered dose equal to 250 mg of neridronic acid, and administering each individual unit dose over a duration of at least 4 hours.

One embodiment of this aspect of the invention is a method, wherein about 300 mg is administered intravenously to the subject in 4 equal individual unit doses on day 1, day 4, day 7, and day 10, to result in a total administered dose equal to 300 mg of neridronic acid, and administering each individual unit dose over a duration of at least 4 hours.

One embodiment of this aspect of the invention is a method, wherein about 400 mg is administered intravenously to the subject in 4 equal individual unit doses on day 1, day 4, day 7, and day 10, to result in a total administered dose equal to 400 mg of neridronic acid, and administering each individual unit dose over a duration of at least 4 hours.

One embodiment of this aspect of the invention is a method, wherein the subject exhibiting the signs and symptoms of CRPS for a duration of 2 years or less and/or exhibiting the signs and symptoms of CRPS in an affected limb and exhibits asymmetry with respect to the contralateral limb experiences a greater decrease in average pain intensity than it would be if the same therapeutically effective amount of neridronic acid was administered intravenously to a control subject exhibiting the signs and symptoms of CRPS for a duration of greater than 2 years, or exhibiting the signs and symptoms of CRPS in an affected limb and not exhibiting asymmetry with respect to the contralateral limb.

One embodiment of this aspect of the invention is a method, wherein the subject exhibiting the signs and symptoms of CRPS for a duration of 2 years or less experiences a change from baseline to week 12 in the average pain intensity score by at least 1 point on the NRS scale.

One embodiment of this aspect of the invention is a method, wherein the subject exhibiting the signs and symptoms of CRPS for a duration of 2 years or less experiences a change from baseline to week 12 in the average pain intensity score by at least 20%.

One embodiment of this aspect of the invention is a method, wherein two or more subjects exhibiting the signs and symptoms of CRPS for a duration of 2 years or less are treated and the proportion of subjects experiencing a change from baseline to week 12 in average pain intensity score by at least 1 point on the NRS scale or by at least 20% of the total number of subjects treated.

The embodiments of the other aspects of the invention apply analogously to the sixth aspect of invention and vice versa.

In a seventh aspect, the invention further relates to neridronic acid for use in a method for the treatment of CRPS or pain associated to CRPS, wherein the effective amount of neridronic acid is administered intravenously in one or as two or more individual unit doses,
wherein each individual unit dose of neridronic acid is administered to the subject by intravenous infusion over a duration of at least three hours, of at least four hours or of at least five hours.

The embodiments of the other aspects of the invention apply analogously to the seventh aspect of invention and vice versa.

In an eighth aspect, the invention further relates to neridronic acid for use in a method for the treatment of CRPS or pain associated to CRPS, wherein the effective amount of neridronic acid is administered intravenously in one or as two or more individual unit doses,
wherein the subject exhibits the sign and symptoms of CRPS for a duration of less than two years.

The embodiments of the other aspects of the invention apply analogously to the eighth aspect of invention and vice versa.

In a ninth aspect, the invention further relates to a method of decreasing average pain intensity in a subject suffering from complex regional pain syndrome (CRPS) or pain associated with CRPS, comprising administering to the subject in need thereof a therapeutically effective amount of neridronic acid,
wherein the therapeutically effective amount of neridronic acid is administered intravenously in one or as two or more individual unit doses,
wherein the subject exhibits the signs and symptoms of CRPS for a duration of 2 years or less.

In one aspect of the ninth aspect, the invention further relates to neridronic acid for use in a method of decreasing average pain intensity in a subject suffering from complex regional pain syndrome (CRPS) or pain associated with CRPS, comprising administering to the subject in need thereof a therapeutically effective amount of neridronic acid,
wherein the therapeutically effective amount of neridronic acid is administered intravenously in one or as two or more individual unit doses,
wherein the subject exhibits the signs and symptoms of CRPS for a duration of 2 years or less.

One embodiment of this aspect of the invention is a method, wherein the subject exhibiting the signs and symptoms of CRPS for a duration of 2 years or less experiences a greater decrease in average pain intensity than it would be if the same therapeutically effective amount of neridronic acid was administered intravenously to a control subject exhibiting the signs and symptoms of CRPS for a duration of greater than 2 years.

One embodiment of this aspect of the invention is a method, wherein the subject exhibiting the signs and symptoms of CRPS for a duration of 2 years or less experiences a change from baseline to week 12 in the average pain intensity score by at least 1 point on the NRS scale.

One embodiment of this aspect of the invention is a method, wherein the subject exhibiting the signs and symptoms of CRPS for a duration of 2 years or less experiences a change from baseline to week 12 in the average pain intensity score by at least 20%.

One embodiment of this aspect of the invention is a method, wherein two or more subjects exhibiting the signs and symptoms of CRPS for a duration of 2 years or less are treated and the proportion of subjects experiencing a change from baseline to week 12 in average pain intensity score by at least 1 point on the NRS scale or by at least 20% of the total number of subjects treated.

One embodiment of this aspect of the invention is a method, wherein about 250 mg of neridronic acid is administered intravenously to the subject exhibiting the signs and symptoms of CRPS for a duration of 2 years or less in 4 equal individual unit doses on day 1, day 3, day 5, and day 7, or on day 1, day 4, day 7, and day 10, or on day 1, day 5, day 9, and day 13, to result in a total administered dose equal to 250 mg of neridronic acid, and administering each individual unit dose by intravenous infusion over a duration of at least 4 hours.

One embodiment of this aspect of the invention is a method, wherein about 300 mg of neridronic acid is administered intravenously to the subject exhibiting the signs and symptoms of CRPS for a duration of 2 years or less in 4 equal individual unit doses on day 1, day 3, day 5, and day 7, or on day 1, day 4, day 7, and day 10, or on day 1, day 5, day 9, and day 13, to result in a total administered dose equal to 300 mg of neridronic acid, and administering each individual unit dose by intravenous infusion over a duration of at least 4 hours.

One embodiment of this aspect of the invention is a method, wherein about 400 mg of neridronic acid is administered intravenously to the subject exhibiting the signs and symptoms of CRPS for a duration of 2 years or less in 4 equal individual unit doses on day 1, day 3, day 5, and day 7, or on day 1, day 4, day 7, and day 10, or on day 1, day 5, day 9, and day 13, to result in a total administered dose equal to 400 mg of neridronic acid, and administering each individual unit dose by intravenous infusion over a duration of at least 4 hours.

The embodiments of the other aspects of the invention apply analogously to the ninth aspect of invention and vice versa.

### EXAMPLES

### Example 1 - Cₘₐₓ and AUC data

Using the data from a Phase II/III trial [NCT02402530 (Clinical Trials Gov No.); 2014-001915-37 (EudraCT No.)] and a Phase I trial for a renal impairment study (single dose of 35 mg neridronic acid for 3 groups wherein each group included 8 subjects with normal, mild or moderate renal impairment corresponding to an estimated glomerular filtration rate (eGFR) of ≥ 90 mL/min/1.73 m²; eGFR = 60-90 mL/min/1.73 m²; and of eGFR = 30-60 mL/min/1.73 m², respectively), a population pharmacokinetic (PopPK) model has been developed to support the clinical development of neridronic acid (Pharmacometric report PP0064P). The PopPK analysis was performed using a nonlinear mixed effects model (Sheiner LB, Beal SL J Pharmacokinet Biopharm 1980; 8 (6): 553-71). Nonlinear mixed-effects models assume variability of the model parameters across individuals, described by statistical distributions.

The PopPK model development and simulations were performed using NONMEM version 7.2 with the features of non-linear model fitting algorithm to estimate parameter values of fixed and random effects of the PopPK model. The tables and graphical presentation of model simulations were conducted using R version 3.0.1 for Linux. The final model included the estimated glomerular filtration rate (eGFR) as a covariate for the compound clearance from the central compartment and the baseline individual weight as a covariate for the volume of the shallow compartment.

The final PopPK model was used to perform simulations to predict the peak concentration in the central compartment (i.e. the plasma) at the end of 100 mg infusions of neridronic acid (C_{max central}) as well as the range of area under the curve of PK concentrations in the central compartment from 0 to 12 days (AUC_{central day1-12}) after the start of the treatment with 100 mg infusions performed at days 1, 4, 7, and 10 (cumulative dose of 400 mg) with an infusion duration of either 2 or 4 hours. A typical weight of 80 kg and a typical eGFR value of 90 mL/min/1.73m² were used. The simulations accounted for the inter-individual variability (IIV) without the residual error. The number of replicates was 1000. The 97.5^{th} percentile of the simulated pharmacokinetic (PK) endpoints was compared between subjects with 2 h infusion and subjects 4 h infusion.

The results are given in Table 1:

**Table 1: Final PopPK model-predicted Cmax central and AUC_{central day1-12} after 2 h or 4 h infusions of 100 mg of neridronic acid (cumulative dose of 400 mg ) at days 1, 4, 7, and 10.**

| Parameter | 2 h infusion | 4 h infusion |
|---|---|---|
| C_{max central} (%CV) ng/mL | 2846 (37%) | 1880 (24%) |
| AUC_{central day1-12} (%CV) ng/mL.h | 49487 (13%) | 49455 (13%) |

| | | |
|---|---|---|
| Cₘₐₓ central and AUC_{central day1-12} were derived based on simulated PK-time profiles over 13 days. | | |

AUC_{central day1-12} = area under the curve of PK concentrations in the central compartment from 0 to 12 days after start of the treatment with 100 mg neridronic acid infusions performed at days 1, 4, 7, and 10 (cumulative dose of 400 mg); Cₘₐₓ central = PK peak concentration in the central compartment at the end of one infusion of 100 mg neridronic acid; %CV: coefficient of variation; PopPK = population pharmacokinetic.

### Example 2 - Duration of signs and symptoms of CRPS

A multi-site, open-label, single-arm, Phase III safety trial of intravenous neridronic acid at a cumulative dose of 400 mg was conducted in subjects with CRPS to investigate the safety and tolerability of intravenous neridronic acid in subjects with CRPS.

The trial product was supplied in glass vials, each containing 111.2 mg sodium neridronate hemihydrate (equivalent to 100 mg neridronic acid) in a total volume of 8 mL. For each infusion, before use, the trial product was diluted in sterile normal saline (0.9% NaCl, e.g., United States Pharmacopeia (USP) grade saline for injection) to a volume of approximately 500 mL and administered by slow intravenous infusion (240 minutes [maximum 260 minutes]) at day 1, day 4, day 7 and day 10, resulting in a total dose of 400 mg neridronic acid.

In addition, the trial provided supportive data on efficacy and long-term effectiveness of neridronic acid of 400 mg. Average, worst (both with a 24-hour recall period) and current pain intensity was measured at the trial visits using an 11-point numerical rating scale on a tablet computer.

The trial included an enrollment period lasting up to 60 days, a treatment period consisting of 4 infusions of neridronic acid over 10 days, and a follow-up period of approximately 50 weeks.

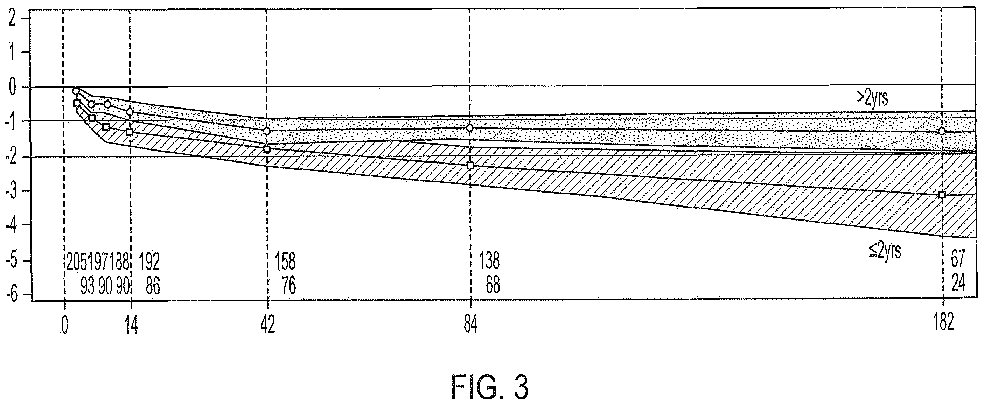

An interim look into the data of this trial was performed when 216 subjects had reached the Week 12 time point, i.e. the trial visit at Day 84 from start of the first infusion. It was found that the mean decrease in average pain intensity from baseline to Day 84 was substantially and clinically relevantly larger in subjects with a duration of CRPS signs and symptoms 2 years or less (≤2 years) than in subjects with a CRPS duration of more than 2 years (>2 years) (Figure 3). The mean (standard deviation) change from baseline to Day 84 was -2.29 (2.193) in subjects with CRPS duration ≤2 years and -1.22 (2.197) in subjects with CRPS duration >2 years. Ninety-five percent confidence intervals for the mean decrease in pain intensity from baseline to Day 84 in both subgroups did not overlap and were [-2.82, -1.76] in subjects with CRPS duration ≤2 years and [-1.59, -0.85] in subjects with CRPS duration >2 years.

## Claims

1. Neridronic acid for use in a method of treating complex regional pain syndrome (CRPS) or pain associated with CRPS, comprising administering to a subject in need thereof a therapeutically effective amount of neridronic acid,
wherein the therapeutically effective amount of neridronic acid is administered intravenously in one or as two or more individual unit doses, wherein each individual unit dose of neridronic acid is administered to the subject by intravenous infusion over a duration of at least 4 hours.

2. Neridronic acid for use in a method according to claim 1, wherein complex regional pain syndrome (CRPS) is complex regional pain syndrome type I (CRPS-I), or wherein complex regional pain syndrome (CRPS) is complex regional pain syndrome type II (CRPS-II).

3. Neridronic acid for use in a method according to claim 1 or 2, wherein the subject in need of treatment is a subject with a reduced or impaired renal function.

4. Neridronic acid for use in a method according to any one of claims 1-3, wherein the therapeutically effective amount of neridronic acid is about 100 mg to about 750 mg.

5. Neridronic acid for use in a method according to any one of claims 1-4, wherein the therapeutically effective amount of neridronic acid is about 150 mg or about 200 mg or about 250 mg or about 300 mg or about 400 mg or about 500 mg, and wherein the neridronic acid is administered intravenously in 4 equal individual unit doses on day 1, day 3, day 5, and day 7, or on day 1, day 4, day 7, and day 10, or on day 1, day 5, day 9, and day 13, to result in a total administered dose equal to the therapeutically effective amount of neridronic acid.

6. Neridronic acid for use in a method according to any one of claims 1-5, wherein the method provides a Cₘₐₓ of neridronic acid of about 100 ng/mL to about 3,500 ng/mL.

7. Neridronic acid for use in a method according to any one of claims 1-6, wherein the method provides an AUC of neridronic acid of about 1,000 ng·hr/mL to about 150,000 ng·hr/mL.

8. Neridronic acid for use in a method according to any one of claims 1-7, wherein the subject exhibits the signs and symptoms of CRPS for a duration of 2 years or less.

9. Neridronic acid for use in a method according to any one of claims 1-8, wherein the treatment for pain associated with CRPS is the relief of pain associated with CRPS as well as all changes, improvements, or reductions in the symptoms of CRPS that improve the quality of life of the treated subject.

10. Neridronic acid for use in a method of decreasing average pain intensity in a subject suffering from complex regional pain syndrome (CRPS) or pain associated with CRPS, comprising administering to the subject in need thereof a therapeutically effective amount of neridronic acid,
wherein the therapeutically effective amount of neridronic acid is administered intravenously in one or as two or more individual unit doses,
wherein the subject exhibits the signs and symptoms of CRPS for a duration of 2 years or less, wherein about 250 mg of neridronic acid is administered intravenously to the subject exhibiting the signs and symptoms of CRPS for a duration of 2 years or less in 4 equal individual unit doses on day 1, day 3, day 5, and day 7, or on day 1, day 4, day 7, and day 10, or on day 1, day 5, day 9, and day 13, to result in a total administered dose equal to 250 mg of neridronic acid, and administering each individual unit dose by intravenous infusion over a duration of at least 4 hours, or
wherein about 300 mg of neridronic acid is administered intravenously to the subject exhibiting the signs and symptoms of CRPS for a duration of 2 years or less in 4 equal individual unit doses on day 1, day 3, day 5, and day 7, or on day 1, day 4, day 7, and day 10, or on day 1, day 5, day 9, and day 13, to result in a total administered dose equal to 300 mg of neridronic acid, and administering each individual unit dose by intravenous infusion over a duration of at least 4 hour, or
wherein about 400 mg of neridronic acid is administered intravenously to the subject exhibiting the signs and symptoms of CRPS for a duration of 2 years or less in 4 equal individual unit doses on day 1, day 3, day 5, and day 7, or on day 1, day 4, day 7, and day 10, or on day 1, day 5, day 9, and day 13, to result in a total administered dose equal to 400 mg of neridronic acid, and administering each individual unit dose by intravenous infusion over a duration of at least 4 hours.

11. Neridronic acid for use in a method of treating complex regional pain syndrome (CRPS) or pain associated with CRPS, comprising administering to a subject in need thereof a therapeutically effective amount of neridronic acid, the method comprising the steps of:
determining whether the subject has reduced or impaired renal function by:
obtaining or having obtained a biological sample from the subject; and
determining or having determined an estimated glomerular filtrate rate (eGFR) on the biological sample using the 2009 Chronic Kidney Disease Epidemiology Collaboration creatinine equation;
if the subject has an eGFR of 60 mL/min/1.73 m² or greater, then administering intravenously about 400 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 400 mg of neridronic acid, and administering each individual unit dose by intravenous infusion over a duration of at least 4 hours;
if the subject has an eGFR of 45 to less than 60 mL/min/1.73 m², then administering intravenously about 300 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 300 mg of neridronic acid, and administering each individual unit dose by intravenous infusion over a duration of at least 4 hours;
if the subject has an eGFR of 30 to less than 45 mL/min/1.73 m², then administering intravenously about 250 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to 250 mg of neridronic acid, and administering each individual unit dose by intravenous infusion over a duration of at least 4 hours.

12. Neridronic acid for use in a method for treating complex regional pain syndrome (CRPS) or pain associated with CRPS, comprising administering to a subject in need thereof a therapeutically effective amount of neridronic acid, the method comprising the steps of:
determining whether the subject exhibits the signs and symptoms of CRPS for a duration of 2 years or less, and/or exhibits the signs and symptoms of CRPS in an affected limb and exhibits asymmetry with respect to the contralateral limb;
if the subject exhibits the signs and symptoms of CRPS for a duration of 2 years or less, and/or exhibits the signs and symptoms of CRPS in an affected limb and exhibits asymmetry with respect to the contralateral limb, then administering intravenously a therapeutically effective amount of about 250 mg, or about 300 mg, or about 400 mg of neridronic acid to the subject in 4 equal individual unit doses on day 1, day 3, day 5, and day 7, or on day 1, day 4, day 7, and day 10, or on day 1, day 5, day 9, and day 13, to result in a total administered dose equal to the therapeutically effective amount of neridronic acid, and administering each individual unit dose by intravenous infusion over a duration of at least 4 hours.

## Patentansprüche

1. Neridronsäure zur Verwendung in einem Verfahren zur Behandlung von komplexem regionalem Schmerzsyndrom (CRPS) oder mit CRPS assoziierten Schmerzen, umfassend das Verabreichen, an ein Subjekt mit Bedarf daran, einer therapeutisch wirksamen Menge an Neridronsäure,
wobei die therapeutisch wirksame Menge an Neridronsäure intravenös in einer oder als zwei oder mehrere individuelle Einheitsdosen verabreicht wird, wobei jede individuelle Einheitsdosis an Neridronsäure an das Subjekt durch intravenöse Infusion über eine Dauer von mindestens 4 Stunden verabreicht wird.

2. Neridronsäure zur Verwendung in einem Verfahren nach Anspruch 1, wobei das komplexe regionale Schmerzsyndrom (CRPS) ein komplexes regionales Schmerzsyndrom Typ I (CRPS-I) ist oder wobei das komplexe regionale Schmerzsyndrom (CRPS) ein komplexes regionales Schmerzsyndrom Typ II (CRPS-II) ist.

3. Neridronsäure zur Verwendung in einem Verfahren nach Anspruch 1 oder 2, wobei das behandlungsbedürftige Subjekt ein Subjekt mit einer reduzierten oder beeinträchtigten Nierenfunktion ist.

4. Neridronsäure zur Verwendung in einem Verfahren nach einem der Ansprüche 1-3, wobei die therapeutisch wirksame Menge an Neridronsäure etwa 100 mg bis etwa 750 mg beträgt.

5. Neridronsäure zur Verwendung in einem Verfahren nach einem der Ansprüche 1-4, wobei die therapeutisch wirksame Menge an Neridronsäure etwa 150 mg oder etwa 200 mg oder etwa 250 mg oder etwa 300 mg oder etwa 400 mg oder etwa 500 mg beträgt und wobei die Neridronsäure intravenös in 4 gleichen individuellen Einheitsdosen an Tag 1, Tag 3, Tag 5 und Tag 7 oder an Tag 1, Tag 4, Tag 7 und Tag 10 oder an Tag 1, Tag 5, Tag 9 und Tag 13 verabreicht wird, um in einer verabreichten Gesamtdosis gleich der therapeutisch wirksamen Menge an Neridronsäure zu resultieren.

6. Neridronsäure zur Verwendung in einem Verfahren nach einem der Ansprüche 1-5, wobei das Verfahren einen Cₘₐₓ von Neridronsäure von etwa 100 ng/ml bis etwa 3.500 ng/ml bereitstellt.

7. Neridronsäure zur Verwendung in einem Verfahren nach einem der Ansprüche 1-6, wobei das Verfahren eine AUC von Neridronsäure von etwa 1.000 ng·h/ml bis etwa 150.000 ng·h/ml bereitstellt.

8. Neridronsäure zur Verwendung in einem Verfahren nach einem der Ansprüche 1-7, wobei das Subjekt die Anzeichen und Symptome von CRPS für eine Dauer von 2 Jahren oder weniger zeigt.

9. Neridronsäure zur Verwendung in einem Verfahren nach einem der Ansprüche 1-8, wobei die Behandlung von mit CRPS assoziierten Schmerzen die Linderung von mit CRPS assoziierten Schmerzen sowie alle Veränderungen, Verbesserungen oder Verringerungen der Symptome von CRPS, die die Lebensqualität des behandelten Subjekts verbessern, ist.

10. Neridronsäure zur Verwendung in einem Verfahren zur Verminderung der durchschnittlichen Schmerzintensität bei einem Subjekt, das an komplexem regionalem Schmerzsyndrom (CRPS) oder mit CRPS assoziierten Schmerzen leidet, umfassend das Verabreichen, an das Subjekt mit Bedarf daran, einer therapeutisch wirksamen Menge an Neridronsäure,
wobei die therapeutisch wirksame Menge an Neridronsäure intravenös in einer oder als zwei oder mehrere individuelle Einheitsdosen verabreicht wird,
wobei das Subjekt die Anzeichen und Symptome von CRPS für eine Dauer von 2 Jahren oder weniger zeigt, wobei etwa 250 mg Neridronsäure intravenös an das Subjekt, das die Anzeichen und Symptome von CRPS für eine Dauer von 2 Jahren oder weniger zeigt, in 4 gleichen individuellen Einheitsdosen an Tag 1, Tag 3, Tag 5 und Tag 7 oder an Tag 1, Tag 4, Tag 7 und Tag 10 oder an Tag 1, Tag 5, Tag 9 und Tag 13 verabreicht werden, um in einer verabreichten Gesamtdosis gleich 250 mg Neridronsäure zu resultieren, und Verabreichen jeder individuellen Einheitsdosis durch intravenöse Infusion über eine Dauer von mindestens 4 Stunden oder
wobei etwa 300 mg Neridronsäure intravenös an das Subjekt, das die Anzeichen und Symptome von CRPS für eine Dauer von 2 Jahren oder weniger zeigt, in 4 gleichen individuellen Einheitsdosen an Tag 1, Tag 3, Tag 5 und Tag 7 oder an Tag 1, Tag 4, Tag 7 und Tag 10 oder an Tag 1, Tag 5, Tag 9 und Tag 13 verabreicht werden, um in einer verabreichten Gesamtdosis gleich 300 mg Neridronsäure zu resultieren, und Verabreichen jeder individuellen Einheitsdosis durch intravenöse Infusion über eine Dauer von mindestens 4 Stunden oder
wobei etwa 400 mg Neridronsäure intravenös an das Subjekt, das die Anzeichen und Symptome von CRPS zeigt, für eine Dauer von 2 Jahren oder weniger zeigt, in 4 gleichen individuellen Einheitsdosen an Tag 1, Tag 3, Tag 5 und Tag 7 oder an Tag 1, Tag 4, Tag 7 und Tag 10 oder an Tag 1, Tag 5, Tag 9 und Tag 13 verabreicht werden, um in einer verabreichten Gesamtdosis gleich 400 mg Neridronsäure zu resultieren, und Verabreichen jeder individuellen Einheitsdosis durch intravenöse Infusion über eine Dauer von mindestens 4 Stunden.

11. Neridronsäure zur Verwendung in einem Verfahren zur Behandlung von komplexem regionalem Schmerzsyndrom (CRPS) oder mit CRPS assoziierten Schmerzen, umfassend das Verabreichen, an ein Subjekt mit Bedarf daran, einer therapeutisch wirksamen Menge an Neridronsäure, wobei das Verfahren die folgenden Schritte umfasst:
Bestimmen, ob das Subjekt eine reduzierte oder beeinträchtigte Nierenfunktion aufweist, durch:
Erhalten oder Erhaltenlassen einer biologischen Probe von dem Subjekt; und
Bestimmen oder Bestimmenlassen einer geschätzten glomerulären Filtratrate (eGFR) an der biologischen Probe unter Verwendung der Kreatinin-Gleichung der Chronic Kidney Disease Epidemiology Collaboration von 2009;
wenn das Subjekt eine eGFR von 60 ml/min/1,73 m² oder größer aufweist, dann intravenöses Verabreichen von etwa 400 mg Neridronsäure an das Subjekt in 4 gleichen individuellen Einheitsdosen an Tag 1, Tag 3, Tag 5 und Tag 7 oder an Tag 1, Tag 4, Tag 7 und Tag 10 oder an Tag 1, Tag 5, Tag 9 und Tag 13, um in einer verabreichten Gesamtdosis gleich 400 mg Neridronsäure zu resultieren, und Verabreichen jeder individuellen Einheitsdosis durch intravenöse Infusion über eine Dauer von mindestens 4 Stunden;
wenn das Subjekt eine eGFR von 45 bis weniger als 60 ml/min/1,73 m² aufweist, dann intravenöses Verabreichen von etwa 300 mg Neridronsäure an das Subjekt in 4 gleichen individuellen Einheitsdosen an Tag 1, Tag 3, Tag 5 und Tag 7 oder an Tag 1, Tag 4, Tag 7 und Tag 10 oder an Tag 1, Tag 5, Tag 9 und Tag 13, um in einer verabreichten Gesamtdosis gleich 300 mg Neridronsäure zu resultieren, und Verabreichen jeder individuellen Einheitsdosis durch intravenöse Infusion über eine Dauer von mindestens 4 Stunden;
wenn das Subjekt eine eGFR von 30 bis weniger als 45 ml/min/1,73 m² aufweist, dann intravenöses Verabreichen von etwa 250 mg Neridronsäure an das Subjekt in 4 gleichen individuellen Einheitsdosen an Tag 1, Tag 3, Tag 5 und Tag 7 oder an Tag 1, Tag 4, Tag 7 und Tag 10 oder an Tag 1, Tag 5, Tag 9 und Tag 13, um in einer verabreichten Gesamtdosis gleich 250 mg Neridronsäure zu resultieren, und Verabreichen jeder individuellen Einheitsdosis durch intravenöse Infusion über eine Dauer von mindestens 4 Stunden.

12. Neridronsäure zur Verwendung in einem Verfahren zur Behandlung von komplexem regionalem Schmerzsyndrom (CRPS) oder mit CRPS assoziierten Schmerzen, umfassend das Verabreichen, an ein Subjekt mit Bedarf daran, einer therapeutisch wirksamen Menge an Neridronsäure, wobei das Verfahren die folgenden Schritte umfasst:
Bestimmen, ob das Subjekt die Anzeichen und Symptome von CRPS für eine Dauer von 2 Jahren oder weniger zeigt und/oder die Anzeichen und Symptome von CRPS in einer betroffenen Extremität zeigt und Asymmetrie in Bezug auf die kontralaterale Extremität zeigt;
wenn das Subjekt die Anzeichen und Symptome von CRPS für eine Dauer von 2 Jahren oder weniger zeigt und/oder die Anzeichen und Symptome von CRPS in einer betroffenen Extremität zeigt und Asymmetrie in Bezug auf die kontralaterale Extremität zeigt, dann intravenöses Verabreichen einer therapeutisch wirksamen Menge von etwa 250 mg oder etwa 300 mg oder etwa 400 mg Neridronsäure an das Subjekt in 4 gleichen individuellen Einheitsdosen an Tag 1, Tag 3, Tag 5 und Tag 7 oder an Tag 1, Tag 4, Tag 7 und Tag 10 oder an Tag 1, Tag 5, Tag 9 und Tag 13, um in einer verabreichten Gesamtdosis gleich der therapeutisch wirksamen Menge an Neridronsäure zu resultieren, und Verabreichen jeder individuellen Einheitsdosis durch intravenöse Infusion über eine Dauer von mindestens 4 Stunden.

## Revendications

1. Acide néridronique destiné à être utilisé dans une méthode de traitement du syndrome douloureux régional complexe (SDRC) ou d'une douleur associée au SDRC_{,} comprenant l'administration d'une quantité thérapeutiquement efficace d'acide néridronique à un sujet en ayant besoin,
dans lequel la quantité thérapeutiquement efficace d'acide néridronique est administrée par voie intraveineuse en une ou deux doses unitaires individuelles ou plus, dans lequel chaque dose unitaire individuelle d'acide néridronique est administrée au sujet par perfusion intraveineuse pendant une durée d'au moins 4 heures.

2. Acide néridronique destiné à être utilisé dans une méthode selon la revendication 1, dans lequel le syndrome douloureux régional complexe (SDRC) est le syndrome douloureux régional complexe de type I (SDRC-I), ou dans lequel le syndrome douloureux régional complexe (SDRC) est le syndrome douloureux régional complexe de type II (SDRC-II).

3. Acide néridronique destiné à être utilisé dans une méthode selon l'une des revendications 1 ou 2, dans lequel le sujet ayant besoin d'un traitement est un sujet présentant une fonction rénale réduite ou altérée.

4. Acide néridronique destiné à être utilisé dans une méthode selon l'une quelconque des revendications 1 à 3, dans lequel la quantité thérapeutiquement efficace d'acide néridronique est d'environ 100 mg à environ 750 mg.

5. Acide néridronique destiné à être utilisé dans une méthode selon l'une quelconque des revendications 1 à 4, dans lequel la quantité thérapeutiquement efficace d'acide néridronique est d'environ 150 mg ou d'environ 200 mg ou d'environ 250 mg ou d'environ 300 mg ou d'environ 400 mg ou d'environ 500 mg, et dans lequel l'acide néridronique est administré par voie intraveineuse en 4 doses unitaires individuelles égales au jour 1, au jour 3, au jour 5 et au jour 7, ou au jour 1, au jour 4, au jour 7 et au jour 10, ou au jour 1, au jour 5, au jour 9 et au jour 13, de manière à obtenir une dose totale administrée égale à la quantité thérapeutiquement efficace d'acide néridronique.

6. Acide néridronique destiné à être utilisé dans une méthode selon l'une quelconque des revendications 1 à 5, dans lequel la méthode fournit une Cₘₐₓ d'acide néridronique d'environ 100 ng/ml à environ 3 500 ng/ml.

7. Acide néridronique destiné à être utilisé dans une méthode selon l'une quelconque des revendications 1 à 6, dans lequel la méthode fournit une ASC d'acide néridronique d'environ 1 000 ng·h/ml à environ 150 000 ng·h/ml.

8. Acide néridronique destiné à être utilisé dans une méthode selon l'une quelconque des revendications 1 à 7, dans lequel le sujet présente les signes et symptômes du SDRC depuis une durée de 2 ans ou moins.

9. Acide néridronique destiné à être utilisé dans une méthode selon l'une quelconque des revendications 1 à 8, dans lequel le traitement de la douleur associée au SDRC consiste à soulager la douleur associée au SDRC ainsi que tous les changements, améliorations ou réductions des symptômes du SDRC qui améliorent la qualité de vie du sujet traité.

10. Acide néridronique destiné à être utilisé dans une méthode de réduction de l'intensité moyenne de la douleur chez un sujet souffrant d'un syndrome douloureux régional complexe (SDRC) ou d'une douleur associée au SDRC, comprenant l'administration d'une quantité thérapeutiquement efficace d'acide néridronique au sujet en ayant besoin,
dans lequel la quantité thérapeutiquement efficace d'acide néridronique est administrée par voie intraveineuse en une ou deux doses unitaires individuelles ou plus,
dans lequel le sujet présente les signes et symptômes du SDRC depuis une durée de 2 ans ou moins,
dans lequel environ 250 mg d'acide néridronique sont administrés par voie intraveineuse au sujet présentant les signes et symptômes du SDRC depuis une durée de 2 ans ou moins en 4 doses unitaires individuelles égales au jour 1, au jour 3, au jour 5 et au jour 7, ou au jour 1, au jour 4, au jour 7 et au jour 10, ou au jour 1, au jour 5, au jour 9 et au jour 13, de manière à obtenir une dose totale administrée égale à 250 mg d'acide néridronique, et chaque dose unitaire individuelle étant administrée par perfusion intraveineuse pendant une durée d'au moins 4 heures, ou
dans lequel environ 300 mg d'acide néridronique sont administrés par voie intraveineuse au sujet présentant les signes et symptômes du SDRC depuis une durée de 2 ans ou moins en 4 doses unitaires individuelles égales au jour 1, au jour 3, au jour 5 et au jour 7, ou au jour 1, au jour 4, au jour 7 et au jour 10, ou au jour 1, au jour 5, au jour 9 et au jour 13, de manière à obtenir une dose totale administrée égale à 300 mg d'acide néridronique, et chaque dose unitaire individuelle étant administrée par perfusion intraveineuse pendant une durée d'au moins 4 heures, ou
dans lequel environ 400 mg d'acide néridronique sont administrés par voie intraveineuse au sujet présentant les signes et symptômes du SDRC depuis une durée de 2 ans ou moins en 4 doses unitaires individuelles égales au jour 1, au jour 3, au jour 5 et au jour 7, ou au jour 1, au jour 4, au jour 7 et au jour 10, ou au jour 1, au jour 5, au jour 9 et au jour 13, de manière à obtenir une dose totale administrée égale à 400 mg d'acide néridronique, et chaque dose unitaire individuelle étant administrée par perfusion intraveineuse pendant une durée d'au moins 4 heures.

11. Acide néridronique destiné à être utilisé dans une méthode de traitement du syndrome douloureux régional complexe (SDRC) ou d'une douleur associée au SDRC, comprenant l'administration d'une quantité thérapeutiquement efficace d'acide néridronique à un sujet en ayant besoin, la méthode comprenant les étapes de :
détermination de l'existence ou non d'une fonction rénale réduite ou altérée chez le sujet par :
l'obtention ou l'obtention préalable d'un échantillon biologique du sujet ; et
la détermination ou la détermination préalable d'un débit de filtration glomérulaire estimé (DFGe) à partir de l'échantillon biologique à l'aide de l'équation de la créatinine de la Chronic Kidney Disease Epidemiology Collaboration de 2009 ;
si le sujet présente un DFGe de 60 ml/min/1,73 m² ou plus, administration par voie intraveineuse d'environ 400 mg d'acide néridronique au sujet en 4 doses unitaires individuelles égales au jour 1, au jour 3, au jour 5 et au jour 7, ou au jour 1, au jour 4, au jour 7 et au jour 10, ou au jour 1, au jour 5, au jour 9 et au jour 13, de manière à obtenir une dose totale administrée égale à 400 mg d'acide néridronique, et administration de chaque dose unitaire individuelle par perfusion intraveineuse pendant une durée d'au moins 4 heures ;
si le sujet présente un DFGe de 45 à moins de 60 ml/min/1,73 m², administration par voie intraveineuse d'environ 300 mg d'acide néridronique au sujet en 4 doses unitaires individuelles égales au jour 1, au jour 3, au jour 5 et au jour 7, ou au jour 1, au jour 4, au jour 7 et au jour 10, ou au jour 1, au jour 5, au jour 9 et au jour 13, de manière à obtenir une dose totale administrée égale à 300 mg d'acide néridronique, et chaque dose unitaire individuelle étant administrée par perfusion intraveineuse pendant une durée d'au moins 4 heures ;
si le sujet présente un DFGe de 30 à moins de 45 ml/min/1,73 m², administration par voie intraveineuse d'environ 250 mg d'acide néridronique au sujet en 4 doses unitaires individuelles égales au jour 1, au jour 3, au jour 5 et au jour 7, ou au jour 1, au jour 4, au jour 7 et au jour 10, ou au jour 1, au jour 5, au jour 9 et au jour 13, de manière à obtenir une dose totale administrée égale à 250 mg d'acide néridronique, et chaque dose unitaire individuelle étant administrée par perfusion intraveineuse pendant une durée d'au moins 4 heures.

12. Acide néridronique destiné à être utilisé dans une méthode de traitement du syndrome douloureux régional complexe (SDRC) ou d'une douleur associée au SDRC, comprenant l'administration d'une quantité thérapeutiquement efficace d'acide néridronique à un sujet en ayant besoin, la méthode comprenant les étapes de :
détermination du fait que le sujet présente ou non les signes et symptômes du SDRC depuis une durée de 2 ans ou moins, et/ou présente les signes et symptômes du SDRC dans un membre atteint et présente une asymétrie par rapport au membre controlatéral ;
si le sujet présente les signes et symptômes du SDRC depuis une durée de 2 ans ou moins, et/ou présente les signes et symptômes du SDRC dans un membre atteint et présente une asymétrie par rapport au membre controlatéral, administration par voie intraveineuse d'une quantité thérapeutiquement efficace d'environ 250 mg, ou d'environ 300 mg, ou d'environ 400 mg d'acide néridronique au sujet en 4 doses unitaires individuelles égales au jour 1, au jour 3, au jour 5 et au jour 7, ou au jour 1, au jour 4, au jour 7 et au jour 10, ou au jour 1, au jour 5, au jour 9 et au jour 13, de manière à obtenir une dose totale administrée égale à la quantité thérapeutiquement efficace d'acide néridronique, et chaque dose unitaire individuelle étant administrée par perfusion intraveineuse pendant une durée d'au moins 4 heures.
